# FASCICULE DE BREVET EUROPEEN

(11) **EP 2 043 588 B1**
(45) Date de publication et mention de la délivrance du brevet: **18.11.2009**
(21) Numéro de dépôt: 07787724.9
(22) Date de dépôt: 19.07.2007
(51) Int. Cl.: A61K 6/087, A61K 6/093

(54) **COMPOSITION DENTAIRE RETICULABLE/POLYMERISABLE PAR VOIE CATIONIQUE**
DURCH EINEN KATIONISCHEN PROZESS VERNETZBARE/POLYMERISIERBARE DENTALZUSAMMENSETZUNG
DENTAL COMPOSITION CROSSLINKABLE/POLYMERIZABLE BY CATIONIC PROCESS

(30) Priorité: 21.07.2006 FR 0606644
(43) Date de publication de la demande: 08.04.2009
(73) Titulaire: Bluestar Silicones France, 69486 Lyon Cedex 03 (FR)
(72) Inventeur: FRANCES, Jean-Marc, 69330 Meyzieu (FR); SCHNEIDER, Sophie, 69360 Ternay (FR)
(86) Numéro de dépôt international: PCT/EP2007/057466
(87) Numéro de publication internationale: WO 2008/009718

(56) Documents cités:
- WO-A-00/19966
- WO-A-00/19967
- WO-A-2005/027857
- WO-A-2005/120439
- WO-A-2005/121200
- US-A1- 2004 186 202

## Description

Le domaine de l'invention est celui des compositions dentaires. Plus précisément, les compositions dentaires mises au point dans le cadre de la présente invention sont utilisables pour la réalisation de prothèses dentaires et pour la restauration dentaire.

Ces compositions dentaires sont classiquement des résines époxy, ou silicones photopolymérisables ou des résines acrylates polymérisables par voie radicalaire. Ces compositions incluent en outre des charges particulaires de renfort (e.g. en silice hydrophobisée), des photoamorceurs et éventuellement des photosensibilisateurs voire d'autres additifs fonctionnels tels que des pigments ou des stabilisants. Une fois mélangées, ces compositions sont mises en forme puis photoréticulées en une masse de structure analogue à celle des dents.

Le fait que la charge soit constituée de particules très fines (≈ 0,01 à 5 µm) et de grande surface spécifique, est un facteur limitant de son taux d'incorporation dans la résine. Cette dernière a en effet une capacité d'absorption limitée. Il en résulte que les taux de charges de telles compositions atteignent rarement plus de 45 % en volume. Cela pénalise donc la fonction de renfort mécanique assignée à la charge articulaire

De plus, les compositions dentaires sont formulées avec des charges de renforcement telles que des verres minéraux ou des silices de combustion de très faible granulométrie dont les silanols de surface et/ou l'eau résiduelle réagissent avec les fonctions cationiques ne permettant pas le stockage des compositions. C'est également le cas lorsque ces verres ou ces silices de combustion sont prétraités par des silanes comme le glycidyloxypropyltriméthoxysilane ou glycidyloxypropyltriéthoxysilane ou encore le méthacryloxypropyltriméthoxysilane. Les charges de renforcement interagissent avec les fonctions réactives des espèces (photo)polymérisables/réticulables et sont ainsi à l'origine de problèmes d'instabilité de la composition dentaire lors de son stockage qui peut atteindre des durées de plusieurs mois. Ce phénomène d'instabilité au stockage est accentué pour les compositions fortement chargées (e.g. taux de charge global≥ 50 %).

Le brevet US-B-6,306,926 concerne des compositions dentaires à base de résines époxy (e.g. UVR^{®} 6105, EPON^{®} 828, GY281^{®}), oxétane, ou vinyléther, entre autres, polymérisables/réticulables, par voie cationique et sous irradiation, et éventuellement des résines (méth)acrylate polymérisables par voie radicalaire. Outre les inducteurs de polymérisation que sont les photoamorceurs cationiques et éventuellement les initiateurs radicalaires selon le cas, ces compositions comprennent une charge minérale microparticulaire, radioopaque, qui est sélectionnée parmi les composés métalliques suivants : oxydes, halogénures, borates phosphates, silicates, carbonates, germanates, tétrafluoroborates, hexatluoro-phosphates, ayant un point isoélectrique inférieur à 7. Cette composition est telle que sa dureté Barcol est d'au moins 10, après 30 min de polymérisation cationique à 25°C.

Ces résines ont l'inconvénient de ne pas être parfaitement transparentes au rayonnement actinique d'activation de la polymérisation par rayonnement UV-visible, ce qui est dommageable à la cinétique réactionnelle et donc limite les possibilités d'obtenir des matériaux photoréticulés très épais.

La demande de brevet FR-A-2 784 025 vise à remédier ce problème en proposant des compositions dentaires à base de résines silicones polymérisablesréticulables, par voie cationique et sous irradiation suivie ou non d'une post-réticulation thermique. Ces résines silicones sont à fonctionnalités oxirane (époxyde, oxétane...) ou vinyléther. De telles compositions comprennent :
- un ou plusieurs polydiméthylsiloxanes réticulables et/ou polymérisables par voie cationique et porteurs à au moins l'une de leurs extrémités des fonctions réactives de formule : 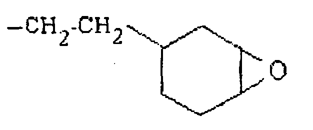
- une quantité efficace d'au moins un amorceur de type borate d'onium: 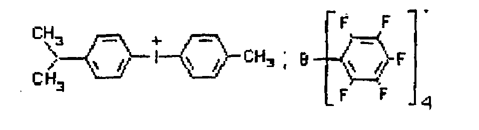
- au moins un photosensibilisateur, et
- au moins une charge dentaire ou de renforcement inerte à base de verres dentaire ou de polyméthacrylate de méthyle ou de silice de combustion éventuellement traitée hexaméthyldisilazane ou polydiméthylsiloxane de surface spécifique 200 m²/g.

Ces compositions dentaires sont destinées à la fabrication de prothèses ou d'appareils dentaires et à la restauration dentaire.

Ces silicones présentent l'avantage par rapport à des résines organiques réticulant par voie cationique d'être très transparents à la lunùère UV-visible et donc de permettre l'obtention de matériaux très épais (plusieurs millimètres d'épaisseur) photoréticulés en très peu de temps (inférieur à une minute) avec une lampe UV émettant dans le domaine du visible >400 nm.

Cependant ces silicones sont formulés avec des charges de renforcement à caractère acide de Lewis ou de Bronsted, telles que des verres broyés ou des silices de combustion de très faible granulométrie, dont les silanols de surface et/ou l'eau résiduelle, réagissent avec les fonctions cationiques. De telles formulations silicones sont donc instables au stockage. De plus, lorsque ces silicones sont fonnulés avec des photosensibilisateurs de type thioxanthone, on observe une grande variation chromatique lors de l'exposition en vue d'une photoréticulation. Ceci se traduit par une coloration rosâtre du produit fini (après exposition) qui n'est pas souhaitable sur le plan esthétique.

On connaît par ailleurs, au travers de la demande de brevet EP-A-1 050 291 des compositions dentaires fortement chargées, présentées comme étant dotées de bonnes propriétés mécaniques et comprenant de 10 à 70 % en volume de charge (e.g. silice de combustion) de granulométrie (Φm) comprise entre 0,05 et 0,5 µm (moins de 50 % en volume de particules de diamètre Φ > 0,50 µm), un monomère acrylique photopolymérisable par voie radicalaire et un dispersant de type ester d'acide phosphorique de formule :

R-[-CO-(CH₂)₅O-]ₙ-PO₃H₂

Cependant, les problèmes de stabilité au stockage des compositions dentaires ne sont pas traités dans cette référence.

Enfin, la demande internationale WO-A-2005121200 décrit des compositions dentaires cationiques et fortement chargées comprenant : au moins un composé (A) réactif par voie cationique ; au moins une charge dentaire (B); éventuellement au moins un dispersant (C) comprenant au moins un polymère ou copolymère organique; au moins un photoamorceur cationique et éventuellement au moins un photosensibilisateur (E), ladite composition étant **caractérisée en ce qu**' au moins une charge dentaire (B) est traitée: par au moins un agent de couplage organosilicé (F) et par au moins un composé (G), ledit agent de couplage organosilicé (F) comprenant au moins une fonction réactive (frA) directement liée à un atome de silicium formant après activation une liaison chimique avec la charge dentaire et au moins une fonction réactive (frB) non directement liée à un atome de silicium dormant après activation une liaison chimique avec une fonction réactive (frC) du composé (G).

Bien que ces compositions constituent une amélioration par rapport à l'art antérieur, il est toutefois souhaitable d'améliorer ces compositions en terme de stabilité au stockage.

Il apparaît donc que l'art antérieur n'apporte pas de solution satisfaisante au problème de stabilité au stockage des compositions dentaires à base de motifs polymérisables par voie cationique sous UV (par exemple des oxiranes).

L'un des objectifs essentiels de la présente invention est donc de remédier à cela en fournissant de nouvelles compositions dentaires à base de motifs polymérisables par voie cationique sous UV (par exemple des oxiranes), ne présentant pas les inconvénients de l'art antérieur sur le plan de la stabilité au stockage.

Un autre objectif essentiel de la présente invention est de fournir de nouvelles compositions dentaires cationiques, polymérisables et/ou réticulables en environnement oral, qui soient non seulement stables au stockage et fortement chargées (e.g. ≥ 50 %) mais qui aient également l'avantage d'être très transparentes a la lumière UV-visible et donc de permettre l'obtention de matériaux très épais (plusieurs millimètres d'épaisseur) photoréticulés en très peu de temps (inférieur à une minute) avec une lampe UV émettant dans le domaine du visible > 400 nm.

Un autre objectif essentiel de la présente invention est de fournir de nouvelles compositions dentaires cationiques, polymérisables et/ou réticulables en environnement oral, qui soient stables, fortement chargées (e.g. ≥ 50 %), faciles à préparer et économiques.

Un autre objectif essentiel de la présente invention est de fournir un nouveau procédé pour traiter une charge de renforcement dentaire, de manière à ce qu'elle puisse répondre aux contraintes exposées ci-dessus lorsqu'elle est utilisée notamment comme charge de renfort dans une composition dentaire.

Ces objectifs, parmi d'autres, sont atteints par la présente invention qui concerne tout d'abord une composition dentaire comprenant :
(1) au moins un composé **(A)** réactif par voie cationique;
(2) au moins une charge dentaire **(B);**
(3) éventuellement au moins une charge dentaire **(B');**
(4) éventuellement au moins un dispersant **(C)** comprenant au moins un polymère ou copolymère organique,
(5) au moins un photoamorceur cationique **(D);** et
(6) éventuellement au moins un photosensibilisateur **(E),**
   ladite composition étant **caractérisée en ce que** la charge dentaire **(B)** est préalablement traitée en milieu solvant par mise en contact avec les ingrédients (a), (b) et (c) suivants:
   (a) au moins un composé **(G)** qui est:
      - un monomère, oligomère ou polymère organique, ou
      - un monomère, oligomère ou polymère organosiloxane,
         ledit composé **(G)** comprenant au moins une fonction réactive **(frC)** oxirane, oxétane, alcényle éther et/ou carbonate,
   (b) au moins un agent de couplage organosilicié **(F)** comprenant au moins une fonction réactive **(frA)** directement liée à un atome de silicium susceptible de réagir avec la charge dentaire **(B)** et au moins une fonction réactive **(frB)** non directement liée à un atome de silicium susceptible de réagir avec une fonction réactive **(frC)** du composé **(G),** et
   (c) au moins un dispersant **(H)** permettant de maintenir la charge dentaire **(B)** sous une forme non agglomérée pendant et/ou après le traitement en milieu solvant.

Il est du mérite des inventeurs d'avoir montré, de manière surprenante et inattendue, que lors d'un pré-traitement en milieu solvant d'une charge dentaire par des espèces chimiques spécifiques selon l'invention, la présence d'un dispersant permet d'éviter la formation d'agglomérat et d'augmenter la stabilité au stockage des compositions dentaires formulées avec ce type de charge. Cette stabilité se traduit par une durée limite d'utilisation de plusieurs mois ou années.

Cette solution technique est d'autant plus intéressante qu'elle est économiquement viable et facile à mettre en oeuvre.

Dans un mode de réalisation préférentiel, le dispersant **(H)** est un polymère ou copolymère organique ou un monodiester d'acide carboxylique.

Dans un deuxième mode de réalisation préféré, le dispersant **(H)** est choisi parmi le groupe constitué par:
- les polymères acryliques éventuellement salifiés par un alkylammonium, les polyesters, les polyéthers, les polyuréthannes, les polyuréthannes modifiés, les polyol-polyacrylates,
- leurs copolymères,
- les monodiesters d'acides carboxyliques,
- les copolymères polyuréthanne/acrylate éventuellement salifiés par un alkylammonium, et
- leurs mélanges.

Des exemples utiles de dispersant **(H)** sont les suivants: les copolymères polyuréthanne/acrylate éventuellement salifiés par un alkylammonium, les copolymères acryliques éventuellement salifiés par un alkylammonium, les monodiesters d'acides carboxyliques, les polyesters, les polyéthers, les polyuréthannes, les polyuréthannes modifiés, les polyol-polyacrylates, leurs copolymères ou leurs mélanges. Les dispersants commercialisés sous la marque DISPERBYK^{®} (de la société Byk) ou SOLSPERSE^{®} (de la société Avecia) conviennent particulièrement à l'invention. On peut citer en particulier et à titre d'exemples, les produits commerciaux: Disperbyk^{®} 164, Disperbyk^{®} 161, Disperbyk^{®}166, Disperbyk^{®}2070, Disperbyk^{®} 9075, Disperbyk^{®} 9076.

On peut citer aussi les dispersants cités dans les brevets suivants :
- le brevet US-B-5,882,393 décrivant des dispersants à base de polyuréthan-nes/imidazoles-acrylates ou époxydes ;
- le brevet US-B-5,425,900 décrivant des dispersants à base de polyuréthannes ;
- le brevet US-B-4,795,796 décrivant des dispersants à base de polyuré-thannes/ polyoxyalkylène-glycolmonoalkyléther;
- la demande de brevet WO-A-99/56864 décrivant des dispersants à base de polyuréthannes/poly(oxyalkylène-carbonyle): dérivés de z-caprolactone et de δ-valérolactone; et
- le brevet EP-B-0 403 197 décrivant des dispersants de type polyol-polyacrylate greffé comprenant un copolymère statistique polyuréthanne/polyvinyli-que/polyacrylate et un polyoxyalkylène polyéther.

Dans un autre mode de réalisation préférentiel :
- la fonction réactive **(frA)** directement liée à un atome de silicium de l'agent de couplage organosilicié **(F)** est une fonction alcoxy, énoxy ou hydroxy,
- la fonction réactive **(frB)** non directement liée à un atome de silicium de l'agent de couplage organosilicié **(F)** est une fonction oxirane, oxétane, hydroxy, acide, anhydride d'acide carboxylique ou diol; et
- la fonction réactive **(frC)** du composé **(G)** est une fonction oxirane, oxétane, alcényle éther ou carbonate.

Selon une variante préférée de l'invention, la composition dentaire selon l'invention comprend au moins une charge dentaire **(B)** traitée par un procédé **(I)** comprenant les étapes suivantes :
a) on mélange en milieu solvant la charge dentaire **(B)** et au moins un agent de couplage organosilicié **(F)** tel que défini ci-dessus,
b) on élimine le solvant par filtration pour obtenir une charge dentaire intermédiaire **(B-1),**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini ci-dessus et en présence d'au moins un dispersant **(H)** tel que défini ci-dessus,
e) on élimine le solvant par filtration pour obtenir une charge dentaire intermédiaire **(B-3),** et
f) la charge dentaire intermédiaire **(B-3)** subie un traitement thermique de manière à terminer la réaction entre la charge dentaire intermédiaire **(B-3)** et le composé **(G)** et ainsi d'obtenir une charge dentaire **(B)** traitée.

Selon une autre variante préférée de l'invention, la composition dentaire selon l'invention comprend au moins une charge dentaire **(B)** traitée par un procédé **(II)** comprenant les étapes suivantes :
a) on mélange en milieu solvant la charge dentaire **(B)** et au moins un agent de couplage organosilicié **(F)** tel que défini ci-dessus,
b) on élimine le solvant par filtration pour obtenir une charge dentaire intermédiaire **(B-1),**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini ci-dessus,
e) on élimine le solvant par filtration pour obtenir une charge dentaire intermédiaire **(B-3),** et
f) la charge dentaire intermédiaire **(B-3)** subie un traitement thermique de manière à terminer la réaction entre la charge dentaire intermédiaire **(B-3)** et le composé **(G)** et ainsi d'obtenir une charge intermédiaire **(B-5),** (idem ligne 6 - page 8)
g) la charge dentaire intermédiaire **(B-5)** est ensuite mélangée en milieu solvant avec au moins un composé **(G)** tel que défini ci-dessus et en présence d'au moins un dispersant **(H)** tel que défini ci-dessus,
h) on élimine le solvant par filtration pour obtenir une charge dentaire intermédiaire **(B-6),** et
i) la charge dentaire intermédiaire **(B-6)** subie un traitement thermique de manière à termine la réaction entre la charge dentaire intermédiaire **(B-6)** et le composé **(G)** et ainsi d'obtenir une charge dentaire **(B)** traitée.

Selon un mode de réalisation préféré, le taux global des charges dentaires **(B)** représente jusqu'à 85 % en poids par rapport au poids total de la composition dentaire et de préférence entre 60 et 80 % en poids.

De manière avantageuse, le traitement de la charge dentaire **(B)** est mis en oeuvre avec jusqu'à 20 % en poids, de préférence entre 1 et 15 % en poids et encore plus préférentiellement entre 2 et 10% en poids du composé **(G)** par rapport au poids total de verre à traiter.
De manière avantageuse, le traitement de la charge dentaire **(B)** est mis en oeuvre avec jusqu'à 20 % en poids, de préférence entre 0,01 et 10 % en poids et encore plus préférentiellement entre 0,1 et 1% en poids du composé **(H)** par rapport au poids total de verre à traiter.
De manière avantageuse, le traitement de la charge dentaire **(B)** est mis en oeuvre avec jusqu'à 20 % en poids, de préférence entre 1 et 15 % en poids et encore plus préférentiellement entre 2 et 10% en poids du composé **(F)** par rapport au poids total de verre à traiter.

L'agent de couplage organosilicié **(F)** a de préférence la formule chimique suivante : 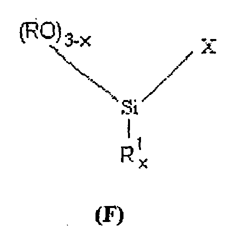 formule dans laquelle:
- R est un hydrogène ou un radical alkyle ou alcényle linéaire ou ramifié en C1 - C4,
- R¹ est un radical alkyle linéaire ou ramifié, ou un radical phényle,
- x est égal à 0, 1 ou 2, et
X étant défini par la formule suivante : 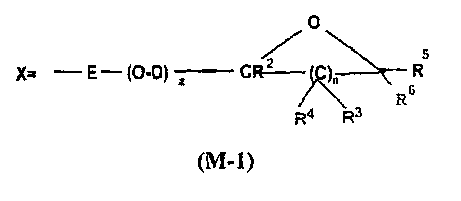 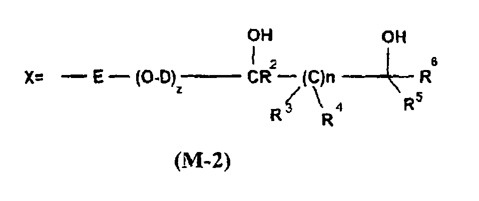 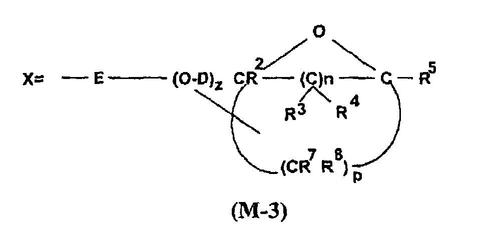 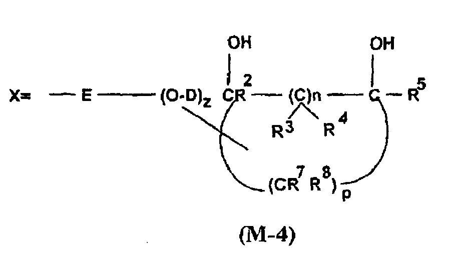 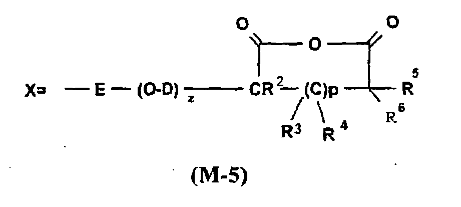 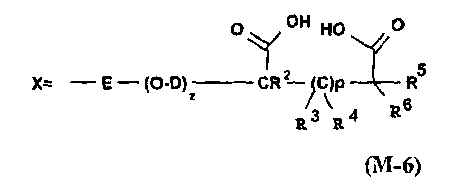 avec :
- E et D qui sont des radicaux identiques ou différents choisis parmi les alkyles en C1-C12 linéaires ou ramifiés,
- z est égal à 0 ou 1 ;
- n est égal à 0 ou 1 ;
- p est égal à 0, 1, 2, 3, 4, 5 ou 6;
- R², R³, R⁴, R⁵, R⁶, R⁷ et R⁸ sont des radicaux, identiques ou différents, représentant un atome d'hydrogène ou un alkyle linéaire ou ramifié en C₁-C₁₂.

Comme agent de couplage organosilicié **(F)** préféré on peut citer les composés suivants : le glycidyloxypropyltriméthoxysilane, le produit d'hydrolyse du glycidyloxypropyltritnéthoxysilane ; le glycidyloxypropyltriéthoxysilane, le produit d'hydrolyse en milieu acide du glycidyloxypropyltriéthoxysilane ; le glycidyloxypropyldiméthoxymethylsilane ou son produit d'hydrolyse , le silane béta-(3,4-époxycyclohexyl)éthyltriéthoxysilane ou son produit d'hydrolyse, le silane béta-(3,4-époxycyclohexyl)éthyltriméthoxysilane ou le produit d'hydrolyse.

Le composé **(G)** est préférentiellement un composé qui est un monomère, un oligomère, un polymère organique ou un organosiloxane comprenant au moins une fonction oxirane, oxétane, alcényle éther et/ou carbonate et encore plus préférentiellement comprenant au moins une fonction oxirane.

D'une manière encore plus préférentielle, le composé **(G)** comprend au moins une fonction choisie parmi le groupe constitué par les structures suivantes **(M-7)** à **(M-12)** :

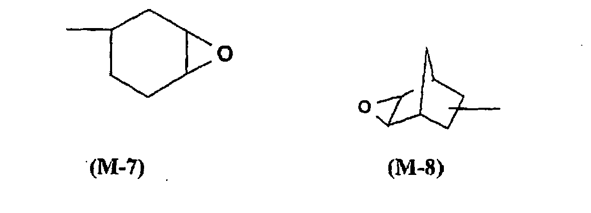

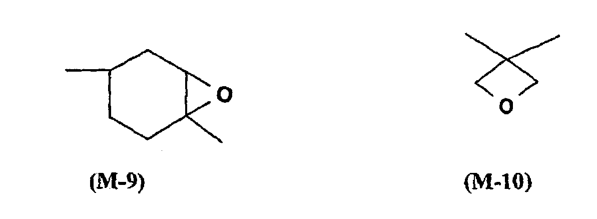

**(M-11)** -(CH₂)₃-O-CH=CH₂

**(M-12)** -(CH₂)₃-O-CH=CH-R"

avec R" représentant un radical alkyle linéaire ou ramifié en C₁-C_{6.}

Selon un autre mode de réalisation préférentiel, le composé **(G)** est un oligomère silicone **(G-1)** ou un polymère silicone **(G-2).** L'oligomère silicone **(G-1)** et le polymère silicone **(G-2)** comprennent :
a) au moins un motif de formule: 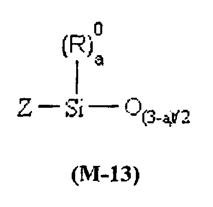 formule dans laquelle :
   - a = 0, 1 ou 2,
   - R⁰, identique ou différent, représente un radical alkyle, cycloalkyle, aryle, vinyle, hydrogéno, alcoxy, de préférence un alkyle inférieur en C₁-C₆,
   - Z, identique ou différent, est un substituant organique comportant au moins une fonction oxirane, alcénylether, oxétane et/ou carbonate, et
b) au moins deux atomes de silicium.

D'une manière avantageuse, l'oligomère silicone **(G-1)** et le polymère silicone **(G-2)** sont choisis parmi le groupe constitué par les composés de formules :
**a)** 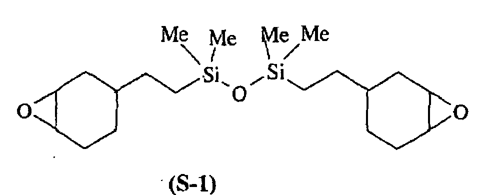
**b)** 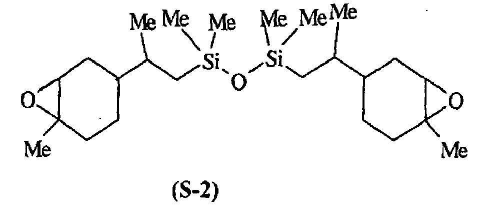
**c)** 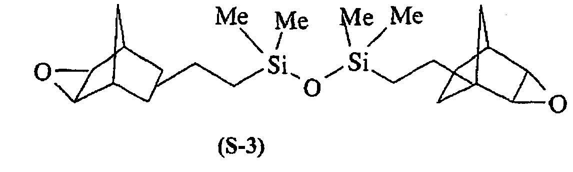
**d)** 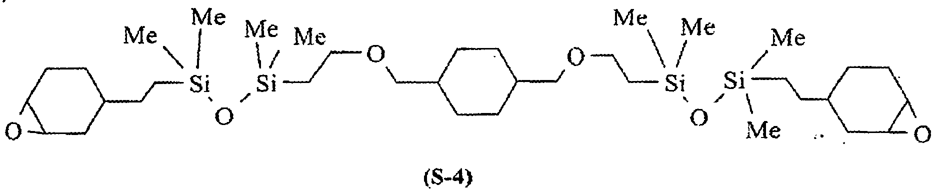
**e)** 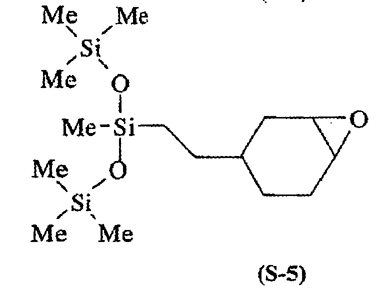
**f)** 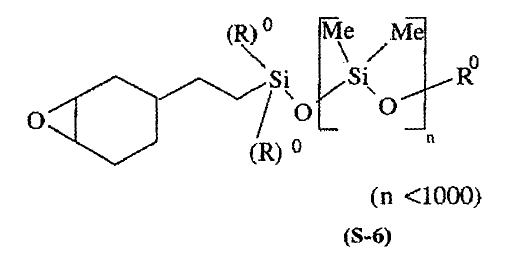
**g)** 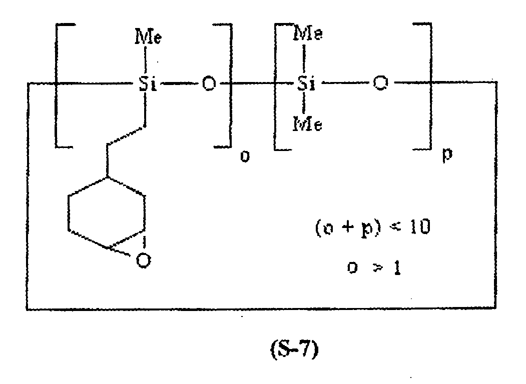
**h)** 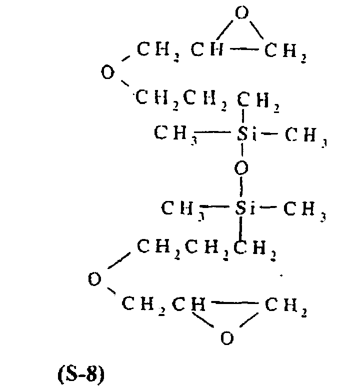
**i)** 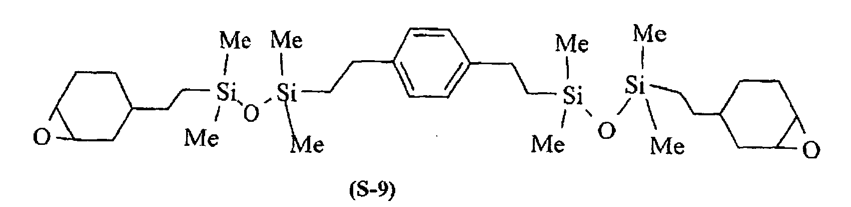
**j)** 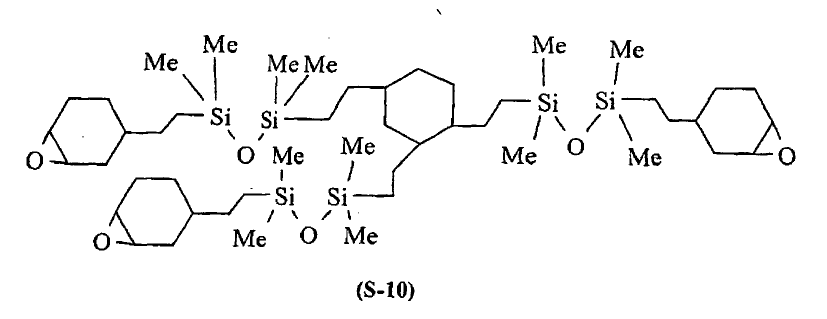
**k)** 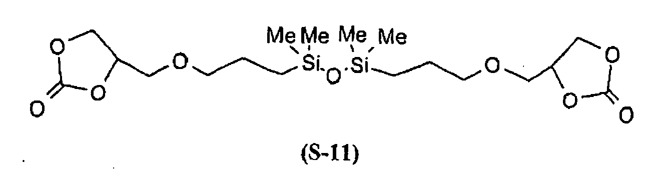
**l)** 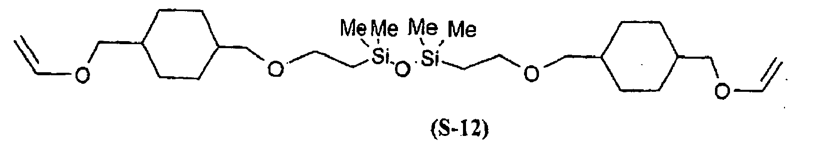
**m)** 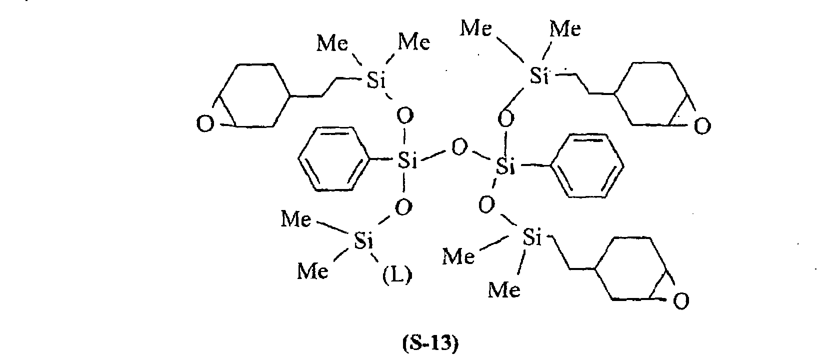 avec L= H ; OH; Me ; phényle; alkyle en C₁-C₁₂; cycloalkyle en C₁-C₆; les groupements; 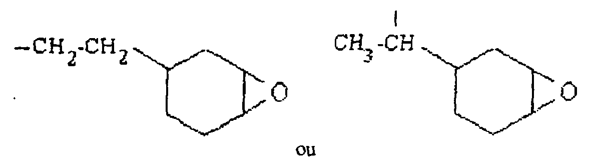
**n)** 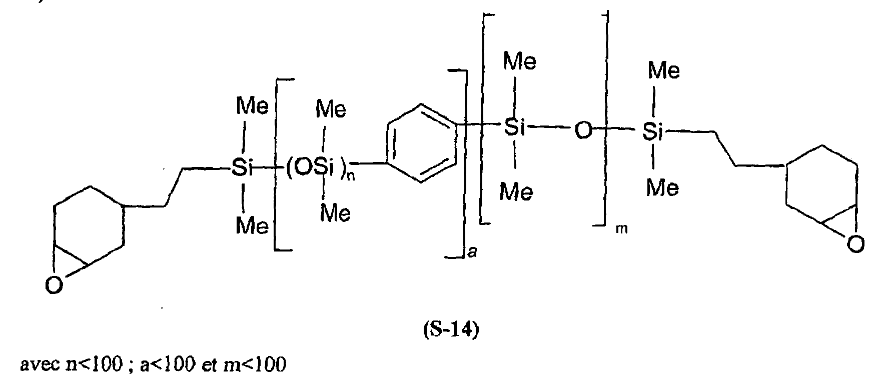
**o)** 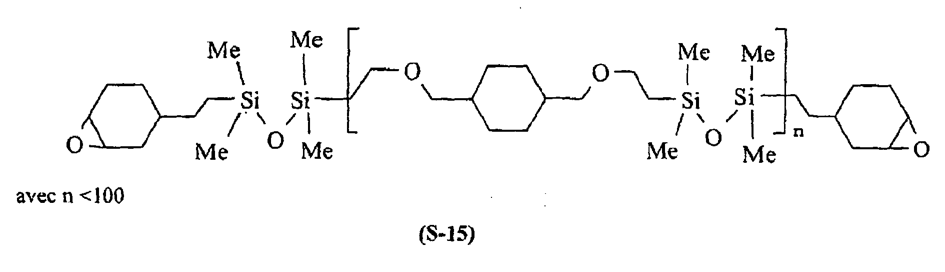 formules dans lesquelles les groupements R, identiques ou différents, représentent un radical alkyle, cycloalkyle ou aryle et de préférence un alkyle inférieure en C₁-C₆. 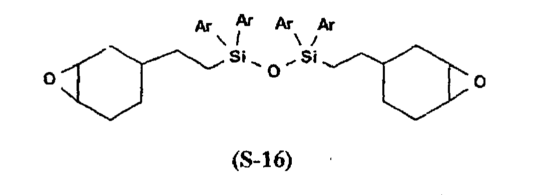 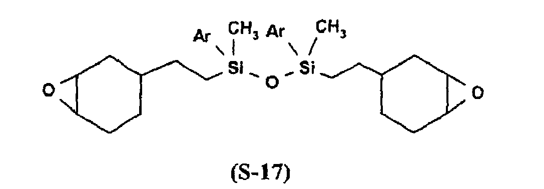 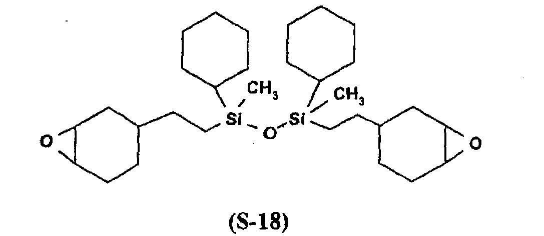 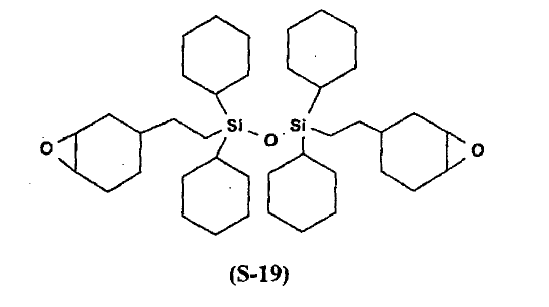 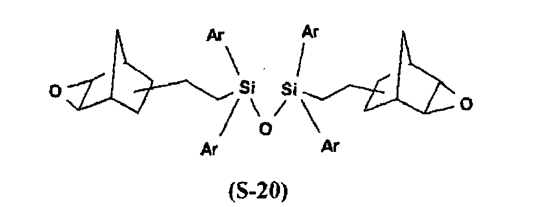 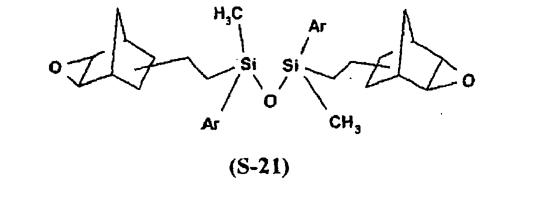 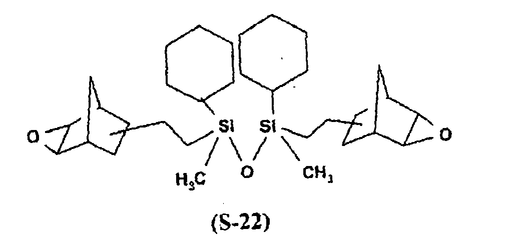 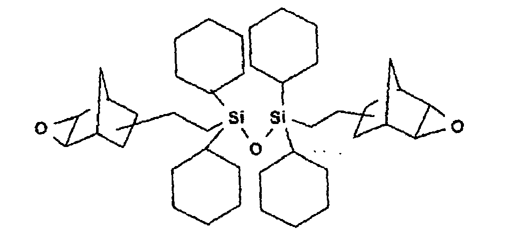 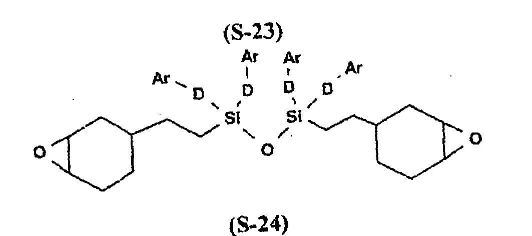 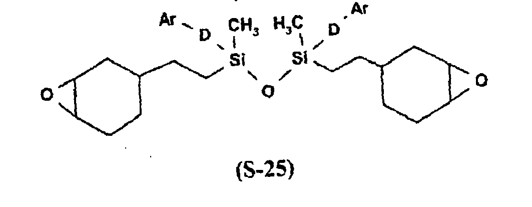 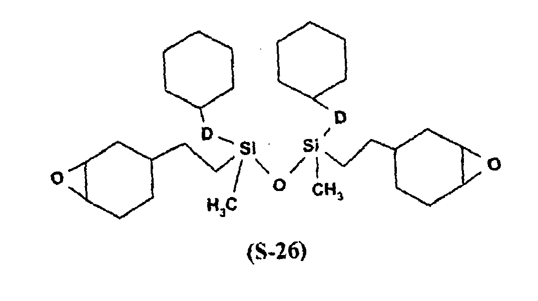 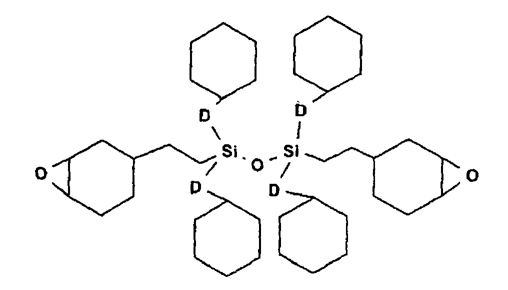 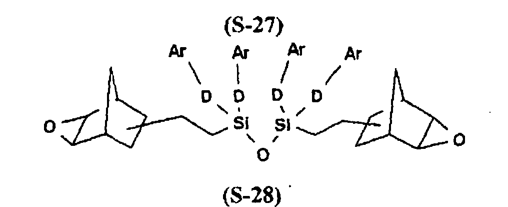 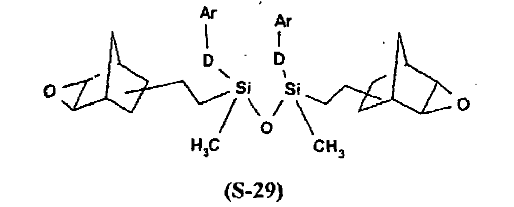 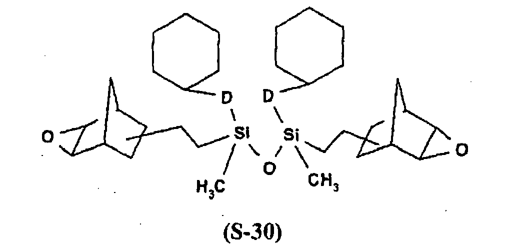 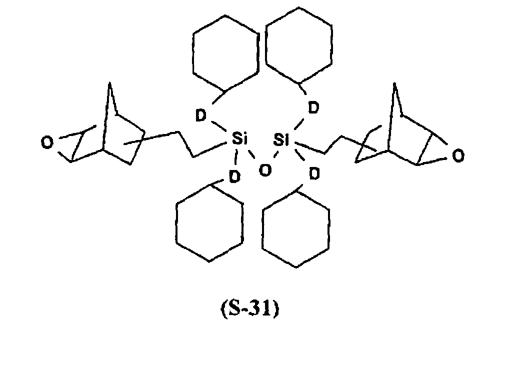 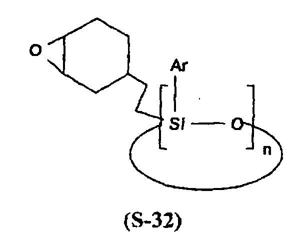 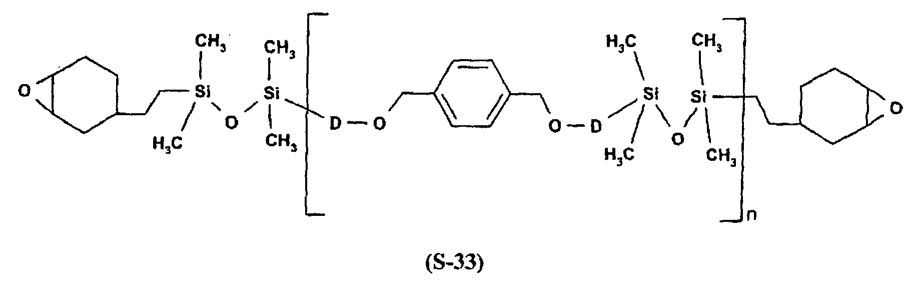 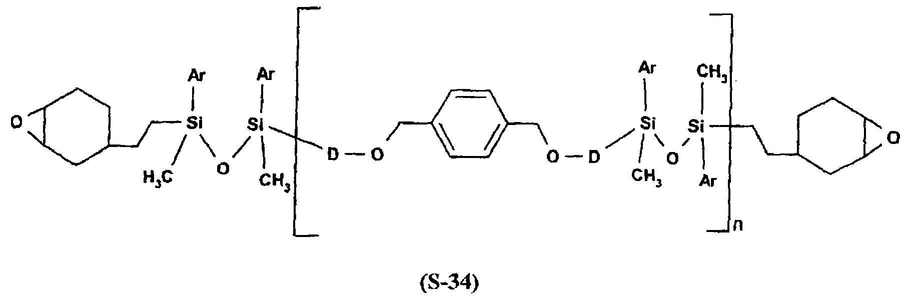 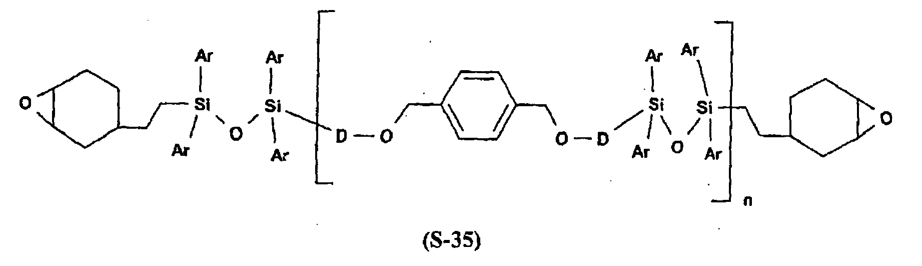 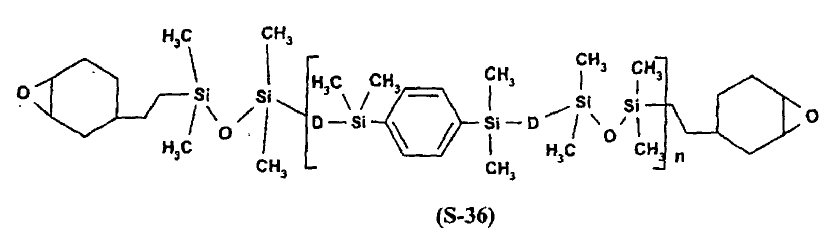 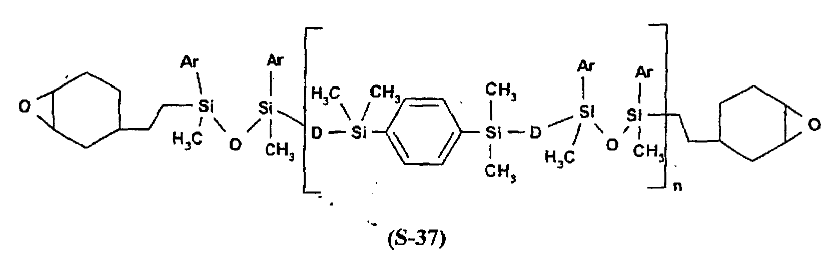 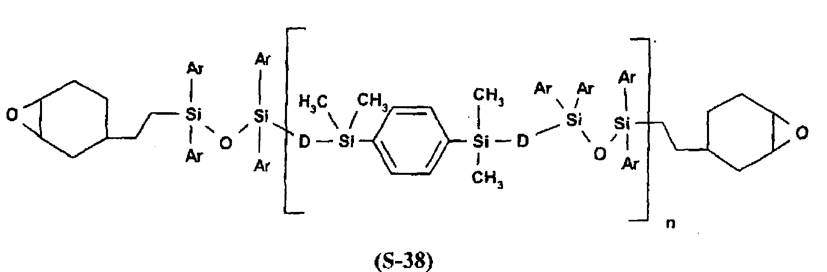 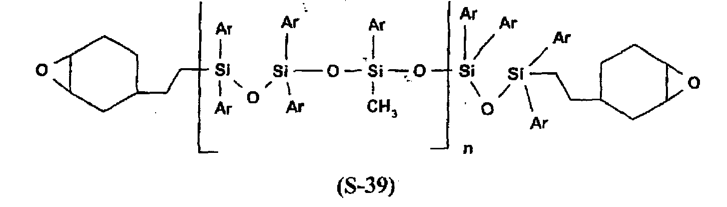 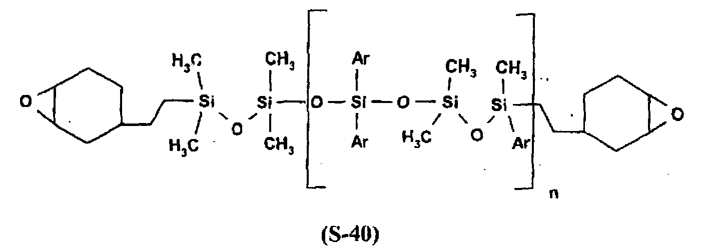 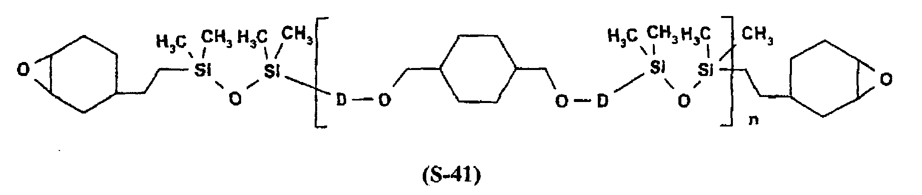 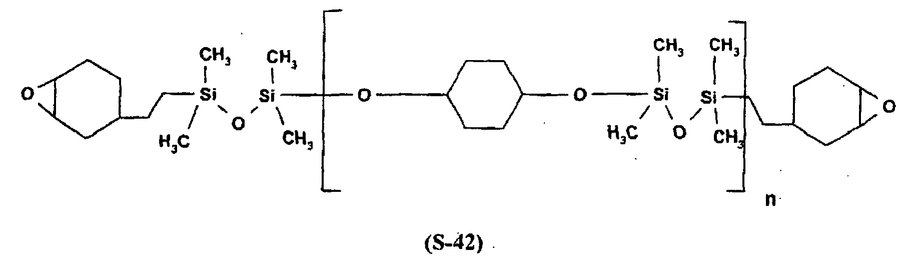 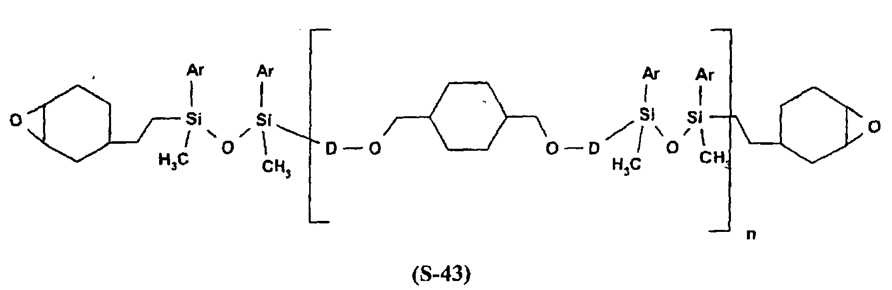 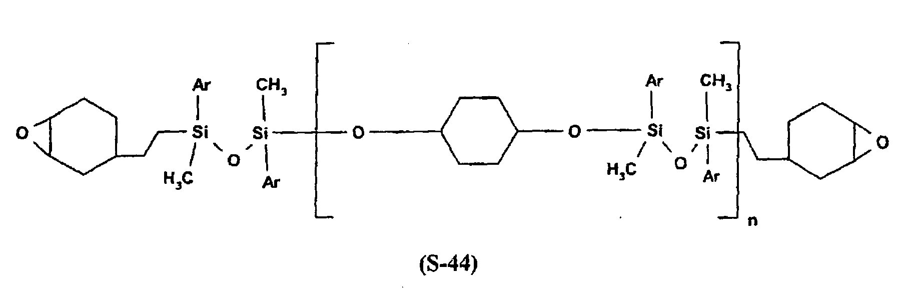 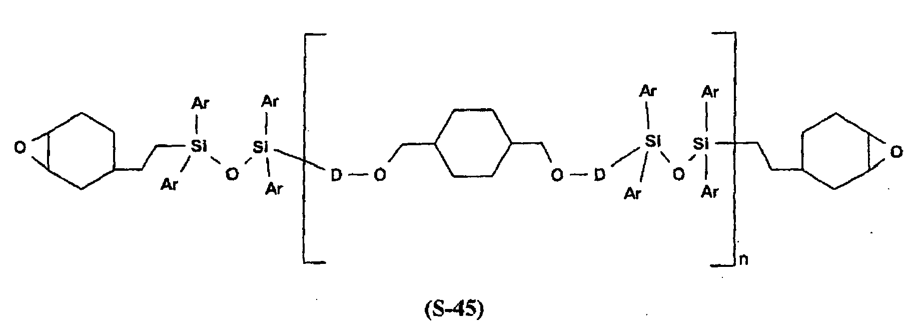 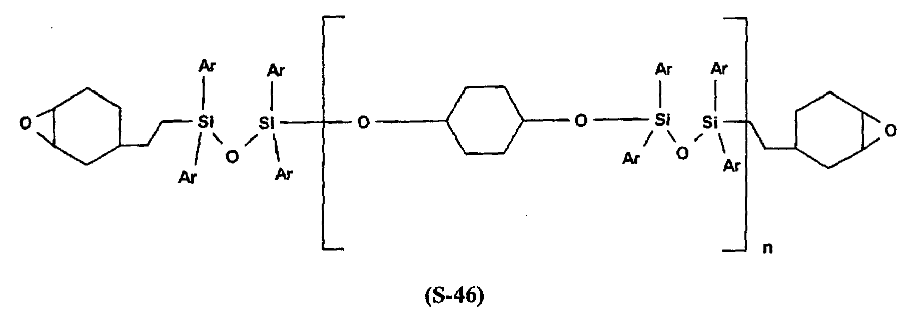 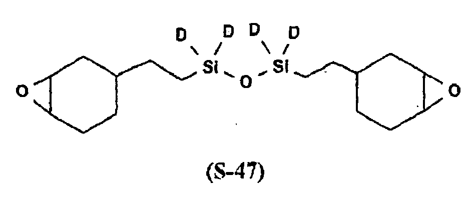 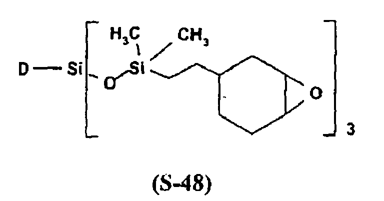 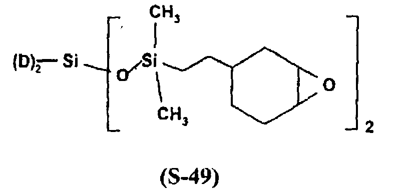 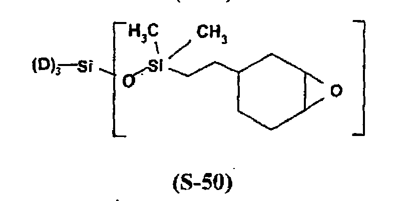 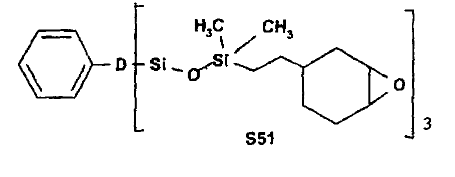 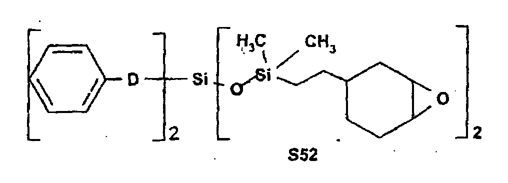 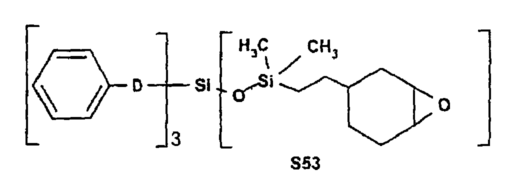 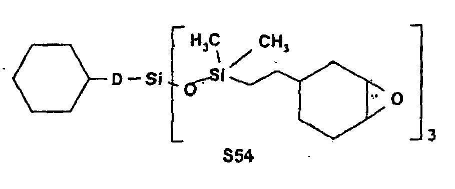 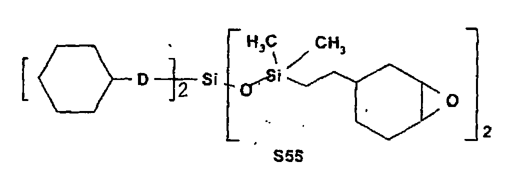 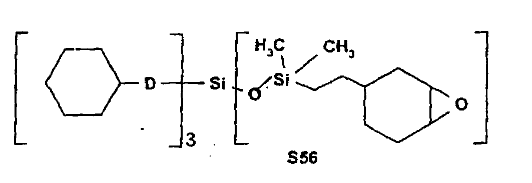 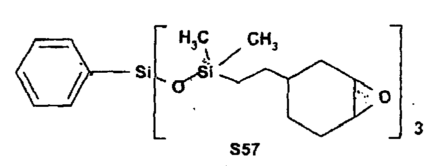 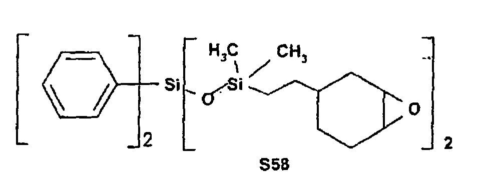 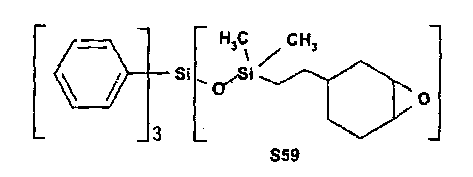 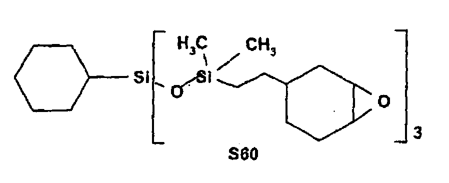 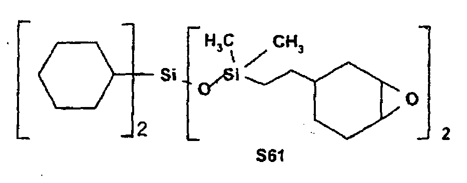 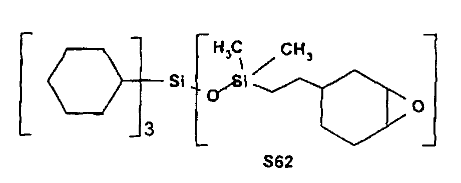 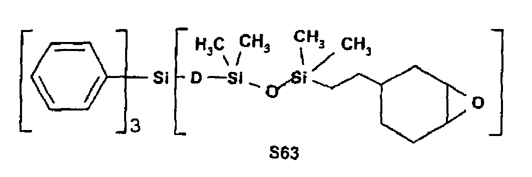 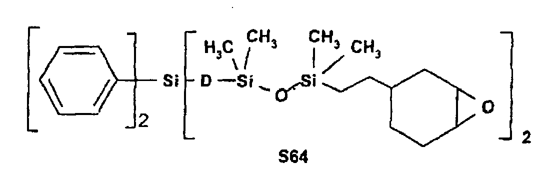 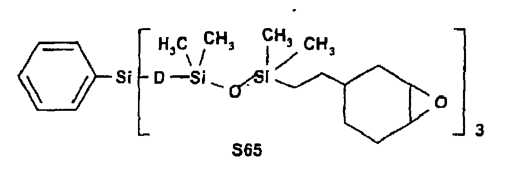 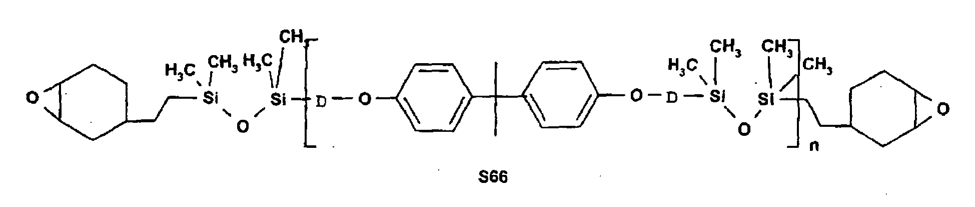 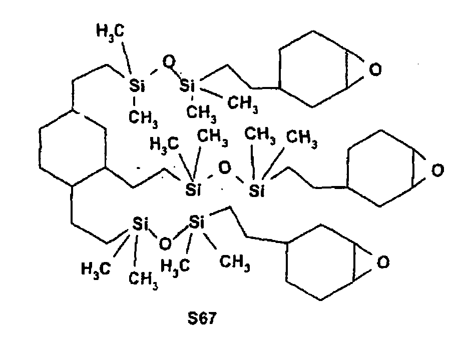 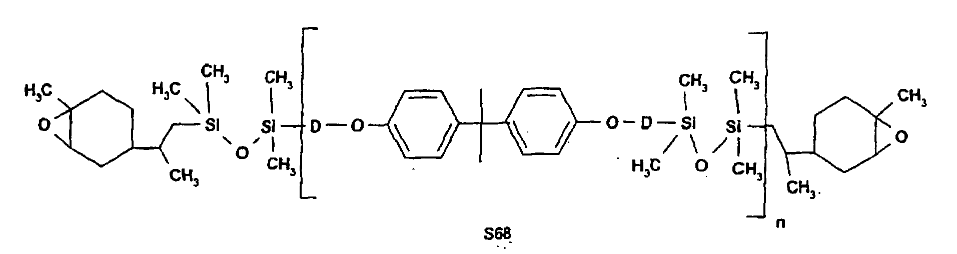 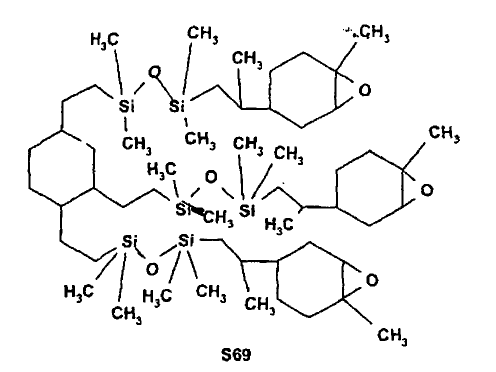 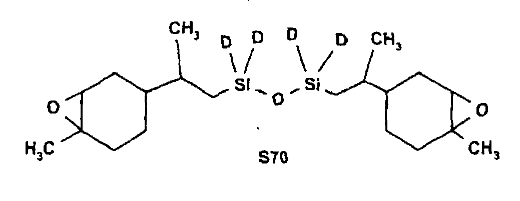 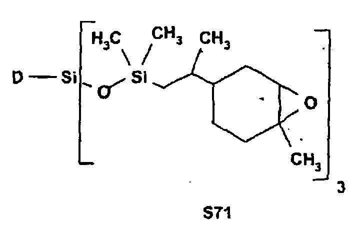 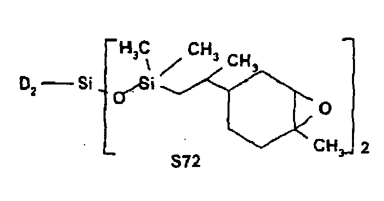 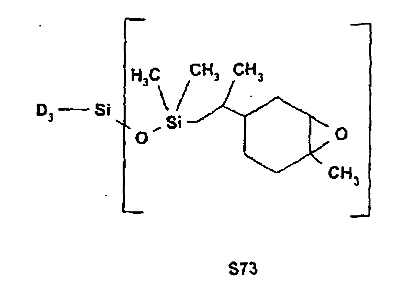 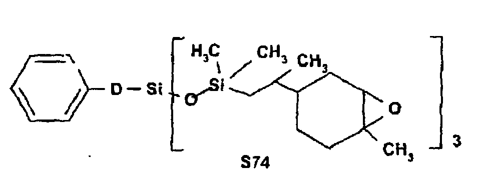 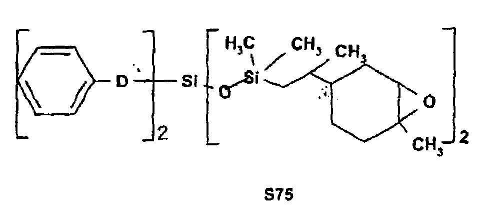 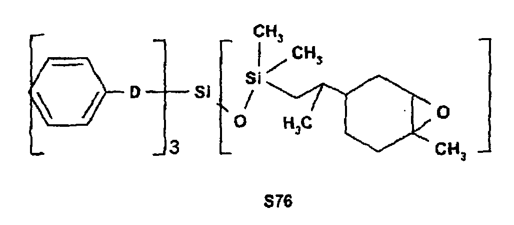 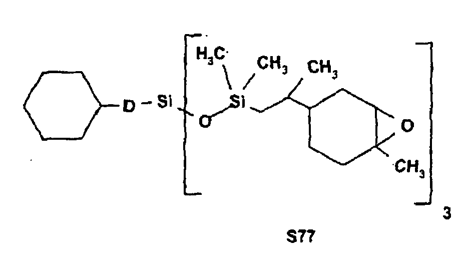 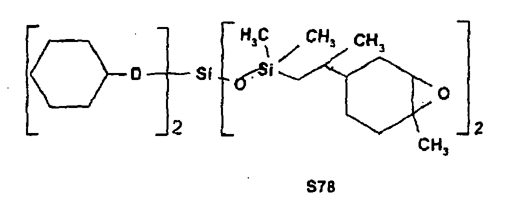 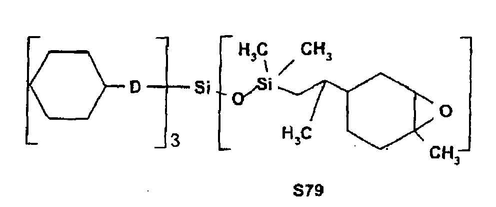 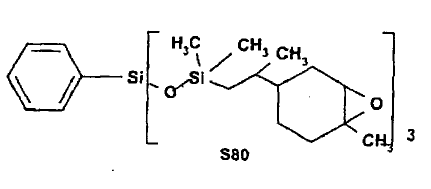 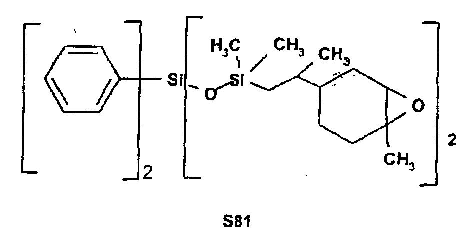 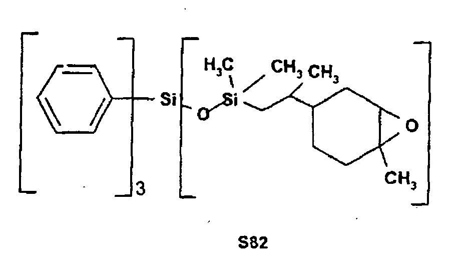 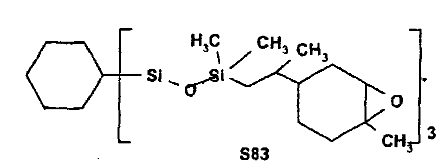 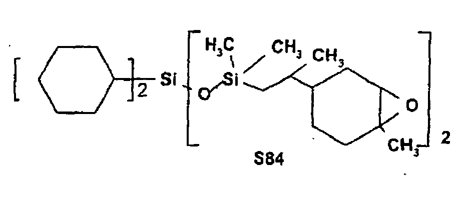 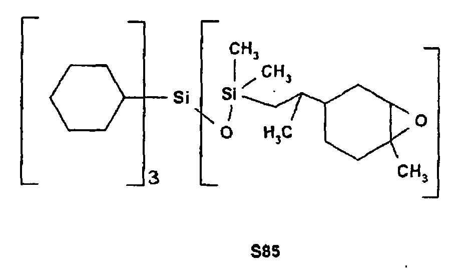 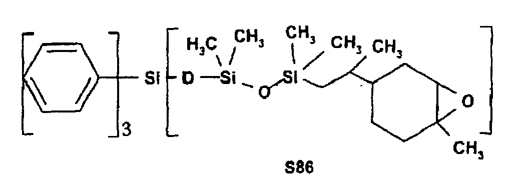 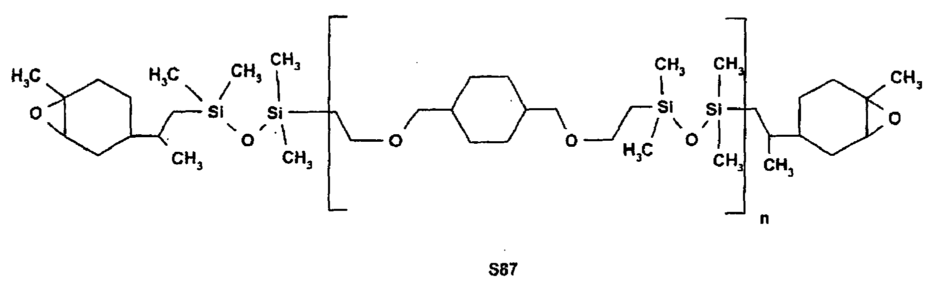 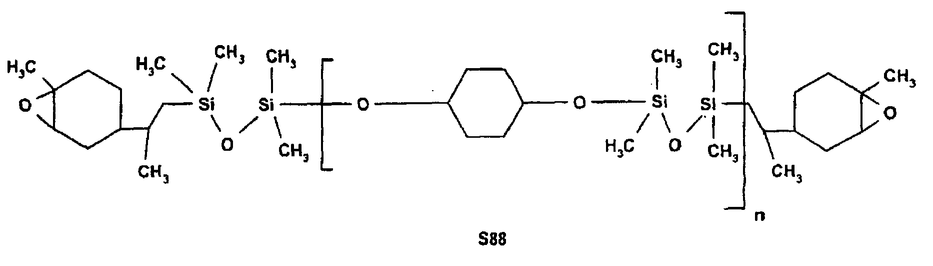 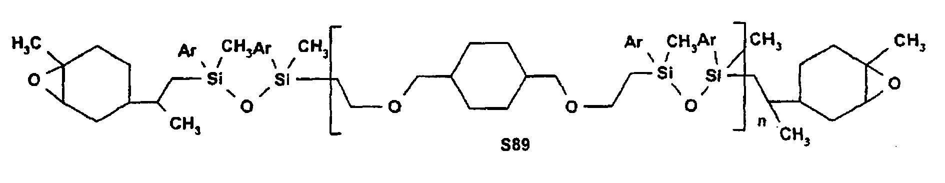 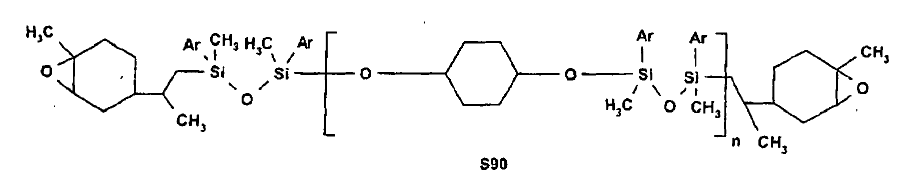 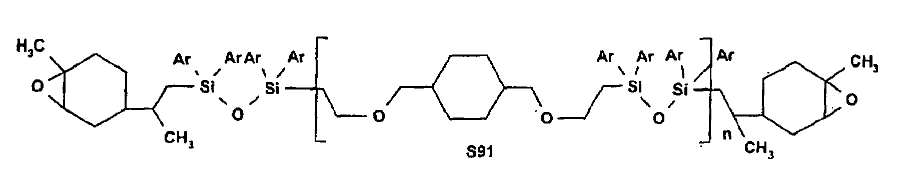 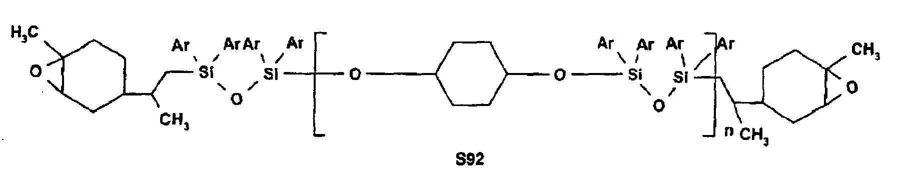 formules dans lesquelles le groupement D est un groupement alkyle en C₁-C₁₂ linéaires ou ramifiés et n est un nombre entier compris entre 1 et 20 (bornes de l'intervalle incluses), avec Ar = groupe aryle.

Selon un autre mode de réalisation préférentiel, le composé **(G)** est un silane **(G-3)** qui est choisi parmi le groupe constitué par les molécules **(S-93)** à **(S-95) :**

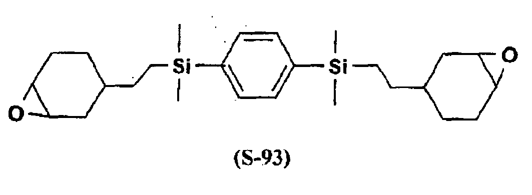

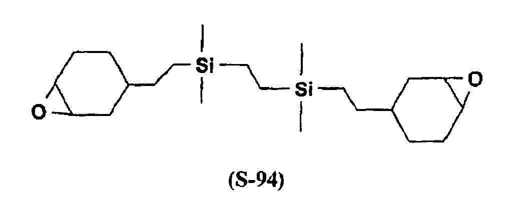

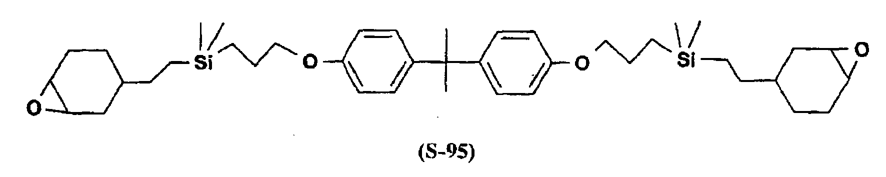

Selon un autre mode de réalisation préférentiel, le composé **(G)** est un composé organique **(G-4)** choisi parmi le groupe constitué par les molécules **(S-96)** à **(S-104) :** 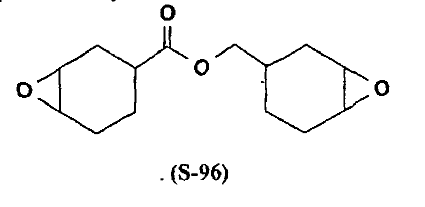 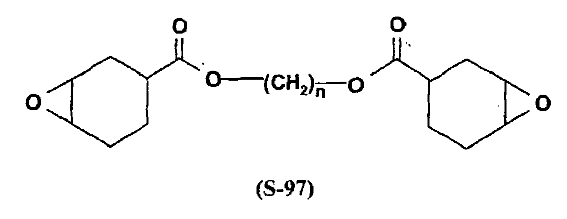 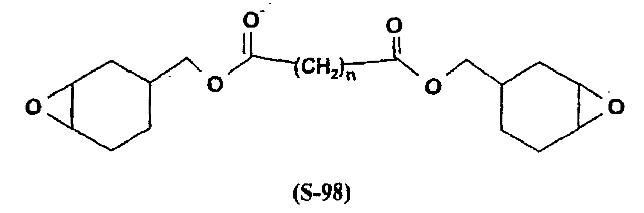 formules dans lesquelles : n est un nombre entier compris entre 1 et 10 (bornes incluses). 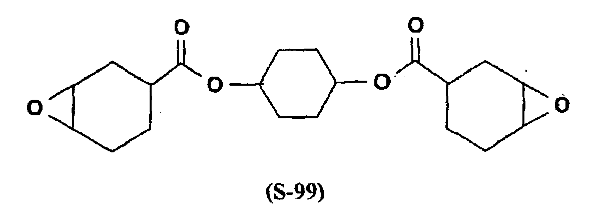 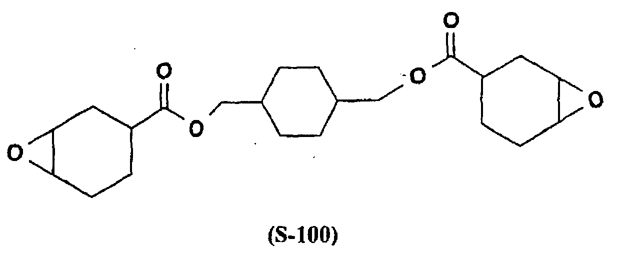 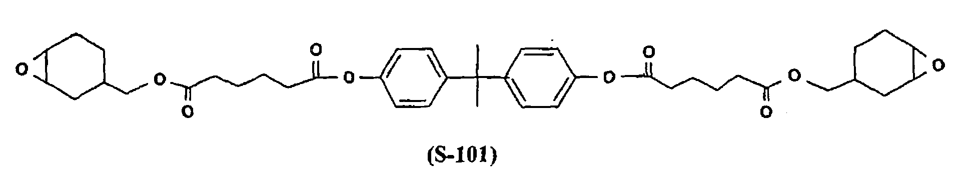 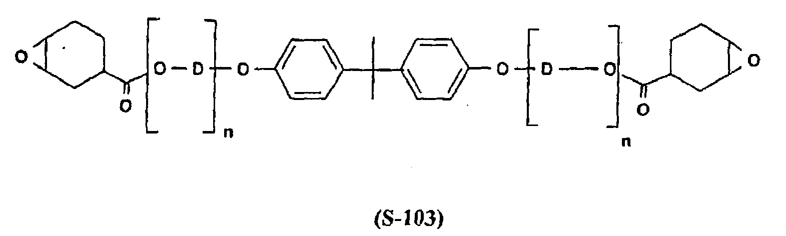 avec n<100 et D= alkyle en C₁-C₁₂ linéaire ou ramifié.

Parmi les molécules de type **(S-103)** on peut choisir la résine UVR6150^{®} commercialisée par la société DOW-CHEMICAL. 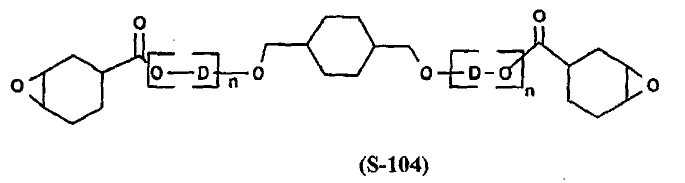 avec n<100 et le groupement D= alkyle en C₁-C₁₂ linéaires ou ramifiés.
Pour les résines de type **(S-104),** celle où n=0 est particulièrement adaptée à l'invention.

En général, l'activation photochimique est réalisée sous rayonnement U.V. Plus particulièrement, on utilise un rayonnement U.V. de longueur d'onde de l'ordre de 200 à 500 nm pour la réalisation de prothèses dentaires et un rayonnement U.V. visible de longueur d'onde supérieure à 400 nm pour la réalisation de matériaux de restauration. Une longueur d'onde supérieure à 400 nm permet la réticulation et/ou polymérisation en environnement oral.

L'activation par voie actinique (photochimique) peut être avantageusement complétée (voire remplacée) par une activation thermique.

De préférence, le composé réactif par voie cationique **(A)** est choisi dans le groupe de monomères et/ou (co)polymères comprenant :
les époxy, les vinyles éthers, les oxétanes, les spiroorthocarbonates, les spiro-orthoesters et leurs associations.

Plus préférentiellement encore, le composé réactif par voie cationique **(A)** est un oligomère silicone **(G-1),** un polymère silicone **(G-2),** un silane **(G-3)** ou un composé organique **(G-4) tel** que défini ci-dessus par le motif **(M-13),** les molécules **(S-1)** à **(S-101).**

Dans la formule **(NI-13),** les fonctions réactives Z particulièrement intéressantes comportent au moins une fonction réactive choisie parmi les radicaux suivants :

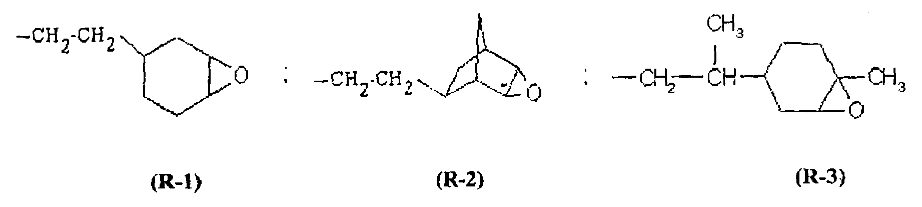

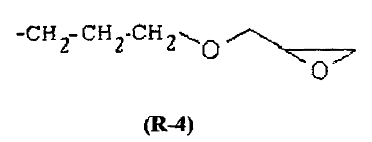

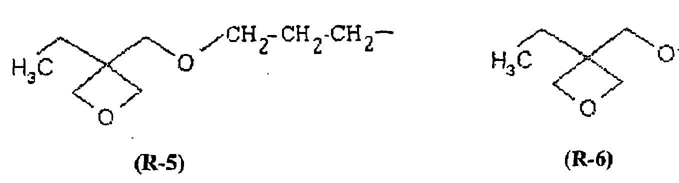

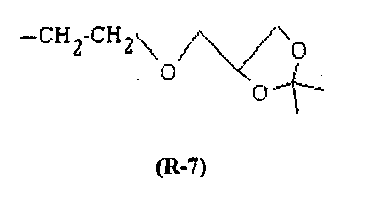

**(R-8) :** -(CH₂)₃-O-CH=CH₂

**(R-9) :** -(CH₂)₃-O-CH=CH-R"

- avec R" représentant un radical alkyle linéaire ou ramifié en C₁-C₆.

Selon une variante avantageuse le composé réactif par voie cationique **(A)** est associé à une résine époxy organique ou oxétane représentant moins de 80% en masse de la fraction. Parmi les résines organiques fonctionnelles choisies on préférera celles dont le pourcentage massique de fonction réactive est inférieur à 20% et de préférence inférieur à 15%. On diminuera d'autant le retrait volumique lors de la polymérisation.

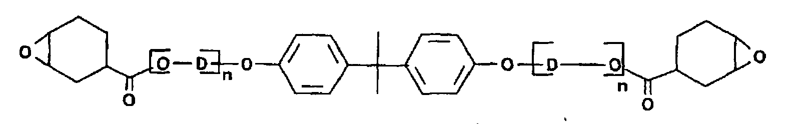

On choisira de préférence les résines de formule **(S-103)** et **(S-104) :**

**(S-103)**

avec n<100 et D= alkyle en C1-C12 linéaires ou ramifiés.
Parmi les résines de type **(S-103)** on peut choisir la résine UVR6150^{®} commercialisée par la société DOW-CHEMICAL. 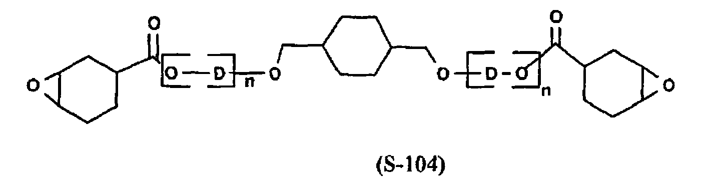 avec n<100 et le groupement D= alkyle en C₁-C₁₂ linéaire ou ramifié.
Pour les résines de type **(S-104),** celle où n=0 est particulièrement adaptée à l'invention.

Différents types de charges dentaires **(B)** et **(B')** sont utilisables pour préparer les compositions selon l'invention. Les charges sont choisies en fonction de l'utilisation finale de la composition dentaire: celles-ci affectent d'importantes propriétés telles que l'apparence, la pénétration du rayonnement U.V., ainsi que les propriétés mécaniques et physiques du matériau obtenu après réticulation et/ou polymérisation de la composition dentaire.

Comme charge de renforcement, on peut utiliser : des charges de silice de pyrogénation traitée ou non, des charges de silice amorphe, du quartz, des verres ou des charges non vitreuses à base d'oxydes de silicium par exemple du type de celles décrites dans US-6297181 (sans baryum), de zirconium, de baryum, de strontium, de calcium, de fluor, d'aluminium, de titane, de zinc, des borosilicates, des aluminosilicates, du talc, des sphérosil, du trifluorure d'yterbium, des charges à base de polymères sous forme de poudre broyée tel que des polytméthacrylates de méthyle inertes ou fonctionnalisés, des polyépoxydes ou des polycarbonates, des whiskers de céramiques (Si-C, Si-O-C, Si-N, Si-N-C, Si-N-C-O) ou des fibres de verre.

A titre d'exemple, on citera :
- des, charges inertes à base de polyméthacrylate de méthyle LUXASELF^{®} de la société UGL utilisables dans le domaine dentaire et pigmentées en rose,
- des charges de silice de combustion traitée hexaméthyldisilazane ou polydiméthylsiloxane de surface spécifique 200 m /g,
- des charges de silice de combustion non traitée (Aerosil-AE200^{®} commercialisée par la société DEGUSSA), et
- des quartz ou verres à base d'oxydes de silicium (charges dentaires de Schott GM27884, G018-066, G018-163 commercialisées par la société Schott)

Selon un mode de réalisation de l'invention, la charge dentaire **(B)** est un verre minéral ou une silice de combustion.

Suivant une caractéristique avantageuse de l'invention,les charges dentaires **(B)** représentent jusqu'à 85 % en poids, de préférence entre 50 et 85 % en poids, encore plus préférentiellement entre 60 et 85 % en poids, par rapport au poids total de la composition dentaire.

A titre d'exemple, le dispersant **(C)** est sélectionné dans le groupe comprenant: les copolymères polyuréthanne/acrylate éventuellement salifiés par un alkylammonium, les copolymères acryliques éventuellement salifiés par un alkylammonium, les monodiesters d'acides carboxyliques, les polyesters, les polyéthers, les polyuréthannes, les polyuréthannes modifiés, les polyol-polyacrylates, leurs copolymères ou leurs mélanges. Les dispersants commercialisés sous la marque DISPERBYK^{®} (de la société Byk) ou SOLSPERSE^{®} (de la société Avecia) conviennent particulièrement à l'invention. On peut citer en particulier et à titre d'exemples, les produits commerciaux: Disperbyk^{®} 164, Disperbyk^{®} 161, Disperbyk^{®} 166, Disperbyk^{®}2070, Disperbyk^{®} 9075, Disperbyk^{®} 9076. On peut citer aussi les dispersants cités dans les brevets suivants :
- le brevet US-B-5,882,393 décrivant des dispersants à base de polyuréthan-nes/imidazoles-acrylates ou époxydes ;
- le brevet US-B-5,425,900 décrivant des dispersants à base de polyuréthannes ;
- le brevet US-B-4,795,796 décrivant des dispersants à base de polyuré-thannes/polyoxyalkylène-glycolmonoalkyléther;
- la demande de brevet WO-A-99/56864 décrivant des dispersants à base de polyuréthannes/poly(oxyalkylène-carbonyle): dérivés de ε-caprolactone et de δ-valérolactone; et
- le brevet EP-B-0 403 197 décrivant des dispersants de type polyol-polyacrylate greffé comprenant un copolymère statistique polyuréthanne/polyvinyli-que/polyacrylate et un polyoxyalkylène polyéther.

Quantitativement parlant, le dispersant **(C)** peut-être présent dans la composition dentaire à raison de 50 ppm à 1 %, de préférence à raison de 100 ppm à 5000 ppm. De préférence, l'indice d'amine du dispersant **(C)** est inférieur ou égal à 60, et plus préférablement encore compris entre 0,1 et 50 mg de potasse par gramme de dispersant **(C).** Avantageusement, l'indice d'acide du dispersant est inférieur ou égal à 200, de préférence inférieur ou égal à 100, et plus préférablement compris entre 1 et 60 mg de potasse par gramme de dispersant.

Les photoamorceurs cationiques **(D)** sont choisis parmi les borates d'onium (pris à eux seuls ou en mélange entre eux) d'un élément des groupes 15 à 17 de la classification périodique [Chem. & Eng. News, vol.63, N° 5, 26 du 4 février 1985] ou d'un complexe organométallique d'un élément des groupes 4 à 10 de la classification périodique [même référence].

Selon un mode préférentiel le photoamorceur cationique **(D)** est de type borate et est choisi parmi ceux dont :
a) l'entité cationique du borate est sélectionnée parmi :
   **(1)** les sels d'onium de formule:

      [(R⁹)ₙ-A-(R¹⁰)ₘ]⁺ **(I)**

      formule dans laquelle :
      - A représente un élément des groupes 15 à 17 tel que par exemple : I, S, Se, P ou N,
      - R⁹ représente un radical aryle carbocyclique ou hétérocyclique en C6-C20, ledit radical hétérocyclique pouvant contenir comme hétéroéléments de l'azote ou du soufre,
      - R¹⁰ représente R⁹ ou un radical alkyle ou alkényle linéaire ou ramifié en C1-C30 ; - lesdits radicaux R⁹ et R¹⁰ étant éventuellement substitués par un groupement alcoxy en C1-C25, alkyle en C₁-C₂₅, nitro, chloro, brome, cyano, carboxy, ester ou mercapto.
      - m et n sont des nombres entiers, avec n + m = v + 1, v étant la valence de l'élément A,
   (2) les sels d'oxoisothiochromauium en l'occurrence ceux décrits dans la demande de brevet WO 90/11303, notamment le sel de sulfonium du 2-éthyl-4-oxoisothiochromanium ou de 2-dodécyl-4-oxoisothio-chromanium et les sels d'oxoisothiochromanium de formule structurale V : 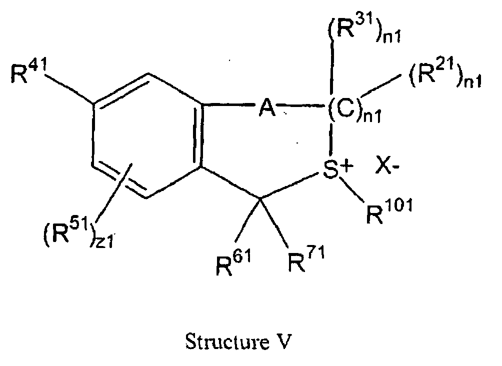 Formule dans laquelle :
      A représente 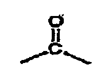 n1 = un entier entre 1 et 3 ;
         z1 = un entier entre 0 et 3 ;
      X représente un groupe de formule (1) M¹Y¹ᵣ₁ ou de formule (2) Q¹,
      où :
      - M¹ = Sb, As, P, B ou Cl,
      - Y¹ représente un halogène (de préférence F ou Cl) ou O et,
      - r1 est un entier compris entre 4 et 6,
      - Q¹représente un acide sulfonique R⁸¹-SO₃ où R⁸¹ est un groupe alkyle ou aryle, ou un groupe alkyle ou aryle substitué par un halogène, de préférence F ou Cl,
      - R¹⁰¹ représente un groupe alkyle ou cycloalkyle, de préférence en C₁-C₂₀, ou un groupe aryle,
      - R²¹ représente un hydrogène, un groupe alkyles, alcényle, cycloalcényle ou cycloalkyle, de préférence en C₁-C₂₀, ou un groupe aryle, tous les R²¹ étant indépendants les uns des autres,
      - R³¹ représente un hydrogène, un groupe alkyle, alcényle, cycloalcényle ou cycloalkyle, de préférence C₁-C₂₀, ou un groupe aryle, tous les R³¹ étant indépendants les uns des autres,
      - R⁴¹ représente un hydrogène, un halogène, un groupe alcényle, par exemple vinyle, cycloalcényle, alkyle, ou cycloalkyle, de préférence en en C₁-C₂₁, un groupe alcoxy ou thioalcoxy, de préférence en en C₁-C₂₀, un groupe poly(alkylènoxyde) avec jusqu'à 10 unités alkylènoxydes terminé par un hydroxyle ou un alkyle (C₁-C₁₂), un groupe aryle, un groupe aryloxy ou thioaryloxy,
      - R⁵¹ représente un halogène, un groupe alcényle, par exemple vinyle, cycloalcényle, alkyle, ou cycloalkyle, de préférence en en C₁-C₂₀, un groupe alcoxy ou thioalcoxy, de préférence en en C₁-C₂₀, un groupe poly(alkylènoxyde) avec jusqu'à 10 unités alkylènoxydes terminé par un hydroxyle ou un alkyle (C₁-C₁₂), un groupe aryle, un groupe aryloxy ou thioaryloxy,
      - R⁶¹ représente un hydrogène, un groupe alkyle, alcényle, cycloalcényle ou cycloalkyle, de préférence en C₁-C₂₀, ou un groupe aryle,
      - R⁷¹ représente un hydrogène, un groupe alkyle, alcényle, cycloalcényle ou cycloalkyle, de préférence en C₁-C₂₀, ou un groupe aryle; et
   (3) les sels organométalliques de formule suivante:

      (L₁L₂L₃M^{)-q} **(II)**

      formule dans laquelle :
      - M représente un métal du groupe 4 à 10, notamment du fer, manganèse, chrome, cobalt,
      - L1 représente 1 ligand lié au métal M par des électrons π, ligand choisi parmi les ligands η³-alkyl, η⁵- cyclopendadiènyl et η⁷- cycloheptratriènyl et les composés η⁶ - aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons n,
      - L2 représente un ligand lié au métal M par des électrons π, ligand choisi parmi les ligands η⁷ - cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π,
      - L3 représente de 0 à 3 ligands identiques ou différents liés au métal M par des électrons σ, ligand(s) choisi(s) parmi CO et NO2+ ; la charge électronique totale q du complexe à laquelle contribuent L1, L2 et L3 et la charge ionique du métal M étant positive et égale à 1 ou 2 ; et
b) les photoamorceurs cationiques (D) dont l'entité anionique borate a pour formule :

   [BXₐ R_{b}]⁻ **(III)**

   formule dans laquelle :
   - a et b sont des nombres entiers allant pour a de 0 à 3 et pour b de 1 à 4 avec a+b=4,
   - les symboles X représentent :
      ^{*} un atome d'halogène (chlore, fluor) avec a = 0 à 3, ou
      ^{*} une fonction OH avec a = 0 à 2,
   - lets symboles R sont identiques ou différents et représentent :
   - un radical phényle substitué par au moins un groupement électroattracteur tel que par exemple OCF₃, CF₃, NO₂ CN, et/ou par au moins 2 atomes d'halogène (fluor tout particulièrement), et ce lorsque l'entité cationique est un onium d'un élément des groupes 15 à 17,
   - un radical phényle substitué par au moins un élément ou un groupement électroattracteur notamment atome d'halogène (fluor tout particulièrement), CF₃, OCF₃, NO₂, CN; et ce lorsque l'entité cationique est un complexe organométallique d'un élément des groupes 4 à 10, ou
   - un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂, CN, quelle que soit l'entité cationique.

Sans que cela ne soit limitatif, sont données ci-après plus de précisions quant aux sous classes de borate d'onium et de borate de sels organométalliques plus particulièrement préférés dans le cadre de l'utilisation conforme à l'invention.

Selon une variante préférée de l'invention, les espèces de l'entité anionique borate qui conviennent tout particulièrement sont les suivantes :
**1'**: [B(C₆F₅)₄]⁻ **5'** : [B(C₆H₃(CF₃)₂)₄]⁻
**2'** : [(C₆F₅)₂BF₂]⁻ **6'** : [B(C₆H₃F₂)₄]⁻
**3'** : [B(C₆H₄CF₃)₄]⁻ **7'** : [C₆F₅BF₃]⁻
**4'** : [B(C₆F₄OCF₃)₄]⁻

Selon une autre variante préférée de l'invention, les sels d'onium de formule **(S-26)** utilisables sont décrits dans de nombreux documents notamment dans les brevets US-A-4 026 705, US-A-4 032 673, US-A-4 069 056, US-A-4 136 102, US-A-4 173 476. Parmi ceux-ci, on privilégiera tout particulièrement les cations suivants :
[(C₈H₁₇)-O-(C₆H₄)-I-C₆H₅)]⁺ ; [C₁₂H₂₅-(C₆H₄)-I-C₆H₅]⁺ ; [(C₈H₁₇-O-(C₆H₄))₂I]⁺
[(C₈H₁₇)-O-(C₆H₄)-I-C₆H₅)]⁺ ; [(C₆H₅)₂S-(C₆H₄)-O-C₈H₁₇]⁺ ;
[CH₃-C₆H₄-I-C₆H₄-CH₂CH(CH₃)₂]⁺ ; [(C₁₂H₂₅-(C₆H₄)-I-(C₆H₄)-CH-(CH₃)₂]⁻
[(C₁₂H₂₅-C₆H₄)₂I]⁺ ; [(C₆H₅)₃S]⁺ ; [CH₃-(C₆H₄)-I-(C₆H₄)-CH(CH₃)₂]⁺
(η5 - cyclopentadiènyle) (η6 - toluène) Fe⁺ ; (η5 - cyclopentadiènyle) (η6 - cumène) Fe⁺,
(η5 - cyclopentadiènyle) (η6 - methyl-1-naphtaléne) Fe⁻;
[(C₆H₅)-S-C₆H₄-S-(C₆H₅)₂]⁺; [(CH₃-(C₆H₄)-I-(C₆H₄)-OC₂H₅]⁺;
[(CₙH₂ₙ₊₁-C₆H₄)₂I]⁺ (avec pour le groupement CₙH₂ₙ₊₁ n= 1 à 18 et est linéaire ou ramifié).

Selon une autre variante préférée, les sels organométalliques (3) de formule **(S-27)** utilisables sont décrits dans les documents US-A-4 973 722, US-A-4 992 572, EP-A-203 829, EP-A-323 584 et EP-A-354 181. Les sels organométalliques plus volontiers retenus selon l'invention sont notamment :
. le (η⁵ - cyclopentadiènyle) (η⁶ - toluène) Fe⁺,
. le (η⁵ - cyclopentadiènyle) (η⁶ - méthyl-1-naphtalène) Fe⁺,
. le (η⁵ - cyclopentadiènyle) (η⁶ - cumène) Fe⁺,
. le bis (η⁶ - mesitylène) Fe⁺,
. le bis (η⁶ - benzène) Cr⁺

En accord avec ces variantes préférées, on peut citer, à titre d'exemples de photoamorceurs du type borates d'onium, les produits suivants :
**(P-16) :** [(C₈H₁₇)-O-C₆H₄-I-C₆H₅)]⁺, [B(C₆F₅)₄]⁻ ;
**(P-17) :** [C₁₂H₂₅-C₆H₄-I-C₆H₅]⁺, [B(C₆F₅)₄]⁻ ;
**(P-18) :** [(C₈H₁₇-O-C₆H₄)₂I]⁺, [B(C₆F₅)₄]⁻ ;
**(P-19) :** [(C₈H₁₇)-O-C₆H₄-I-C₆H₅)]⁺, [B(C₆F₅)₄]⁻ ;
**(P-20) :** [(C₆H₅)₂S-C₆H₄-O-C₈H₁₇]⁺, [B(C₆H₄CF₃)₄]⁻ ;
**(P-21) :** [(C₁₂H₂₅-C₆H₄)₂I]⁺, [B(C₆F₅)₄]⁻ ;
**(P-22) :** [CH₃-C₆H₄-I-C₆H₄-CH(CH₃)₂]⁺, [B(C₆F₅)₄]⁻ ;
**(P-23) :** (η5 - cyclopentadiènyle) (η6 - toluène) Fe⁺, [B(C₆F₅)₄]⁻ ;
**(P-24)** : (η5 - cyclopentadiènyle) (η6 - methyl-1-naphtalène) Fe⁺, [B(C₆F₅)₄]⁻ ;
**(P-25)** : (η5 - cyclopentadiènyle) (η6 - cumène) Fe⁺, [B(C₆F₅)₄]⁻ ;
**(P-26)** : [C₁₂H₂₅-C₆H₄)₂I]⁺, [B(C₆H₃(CF₃)₂]⁻ ;
**(P-27) :** [CH₃-C₆H₄-I-C₆H₄-CH₂CH(CH₃)₂]⁺, [B(C₆F₅)4]⁻ ;
**(P-28)** : [CH₃-C₆H₄-I-C₆H₄-CH₂CH(CH₃)₂]⁺, [B(C₆H₃(CF₃)₂)₄]⁻; et
**(P-29) :** [CH₃-C₆H₄-I-C₆H₄-CH(CH₃)₂]⁺, [B(C₆H₃(CF₃)₂)₄)⁻.

Comme autre référence littéraire pour définir les borates d'onium **(1)** et **(2)** et les borates de sels organométalliques **(3),** on peut citer l'ensemble du contenu des demandes de brevet EP-A-0 562 897 et EP-A-0 562 922. Ce contenu est intégralement incorporé par référence dans le présent exposé.

Comme autre exemple de sel d'onium utilisable comme photoamorceur, on peut citer ceux divulgués dans les brevets américains US 4 138 255 et US 4 310 469. On peut également utiliser d'autres photoamorceurs cationiques, par exemple:
- les sels d'iodonium hexafluorophosphate ou hexafluoro-antimonate, tels que :
- [CH₃-[(C₆H₄)-1-[(C₆H₄)-CH(CH₃)₂]⁺ [PF₆]⁻;
- [CH₃-(C₆H₄)-I-(C₆H₄)-CH₂CH(CH₃)₂]⁺ [PF₆]⁻;
- [(C₁₂H₂₅-C₆H₄)₂I]⁺[PF₆]⁻ ou
- les sels de ferrocénium de ces différents anions.

Le photosensibilisateur contenu au sein de la composition dentaire selon l'invention peut être de nature très variée. Dans le cadre de l'invention celui-ci répond notamment à l'une des formule **(IV)** à **(XXIV)** suivantes : 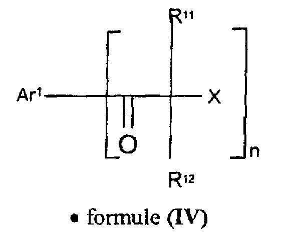 dans laquelle n=1 1 ou 2 :
- lorsque n = 1, **Ar¹** représente un radical aryle contenant de 6 à 18 atomes de carbone, un radical tétrahydronaphtyle, thiényle, pyridyle ou furyle ou un radical phényle porteur d'un ou plusieurs substituants choisis dans le groupe constitué de F, Cl, Br, CN, OH, les alkyles linéaires ou ramifiés en C₁-C₁₂, -CF³, -OR¹³, -OPhényle, -SR¹³, -SPhényle, -SO₂Phényle, -COOR¹³, -O-(CH₂-CH=CH₂), -O(CH₂H₄-O)ₘ-H, -O(C₃H₆O)ₘ-H, m étant compris entre 1 et 100,
- lorsque n = 2, **Ar¹** représente un radical arylène en C₆-C₁₂ ou un radical phénylène-T-phénylène, où T représente -O-, -S-, -SO₂- ou -CH₂-,
- **X** représente un groupe -OR¹⁴ ou -OSiR¹⁵(R¹⁶)₂ ou forme, avec R¹¹, un groupe -O-CH(R¹⁷)-,
- **R¹¹** représente un radical alkyle linéaire ou ramifié en C₁-C₈ non substitué ou porteur d'un groupe -OH, -OR¹³, acyloxy en C₂-C₈, -CF³, ou -CN, un radical alcényle en C₃ ou C₄, un radical aryle en C₆ à C_{18_}, un radical phénylalkyle en C₇ à C₉,
- **R¹²** a l'une des significations données pour R¹¹ ou représente un radical
- CH₂CH₂R¹⁸, ou encore forme avec R¹¹, un radical alkylène en C₂-C₈ ou un radical oxaalkylène ou aza-alkylène en C₃-C₉,
- **R¹³** représente un radical alkyle inférieur contenant de 1 à 12 atomes de carbone,
- **R¹⁴** représente un atome d'hydrogène, un radical alkyle en C₁-C₁₂, un radical alkyle en C₂-C₆ porteur d'un groupe -OH, -OR¹³ ou CN, un radical alcényle en C₃-C₆, un radical cyclohexyle ou benzyle, un radical phényle éventuellement substitué par un atome de chlore ou un radical alkyle linéaire ou ramifié en C₁-C₁₂, ou un radical tétrahydropyrannyle-2,
- **R¹⁵** et **R¹⁶** sont identiques ou différents et représentent chacun un radical alkyle en C₁-C₄ ou un radical phényle,
- **R¹⁷** représente un atome d'hydrogène, un radical alkyle en C₁-C₈ ou un radical phényle,
- **R¹⁸** représente un radical -CONH₂, -CONHR¹³, -CON(R¹³)₂, -P(O)(OR¹³)₂ ou pyridyle-2 ; 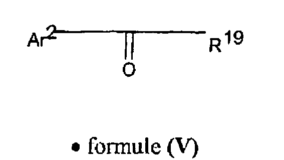 dans laquelle :
   - **Ar²** a la même signification que Ar¹ de la formule **(IV)** dans le cas où n = 1,
   - **R¹⁹** représente un radical choisi panni le groupe constitué d'un radical **Ar²,** un radical alkyle linéaire ou ramifié en C₁-C₁₂, un radical cycloalkyle en C₆-C₁₂ et un radical cycloalkyle formant un cycle en C₆-C₁₂ avec le carbone de la cétone ou un carbone du radical **Ar²,** ces radicaux pouvant être substitués par un ou plusieurs substituants choisis dans le groupe constitué de -F, -Cl, -Br, -CN, -OH, -CF₃, -OR¹³, -SR¹³, -COOR¹³, les radicaux alkyles linéaires ou ramifiés en C₁-C₁₂ porteurs éventuellement d'un groupe -OH, -OR¹³ et/ou -CN, et les radicaux alcényles linéaires ou ramifiés en C₁-C₈ ; 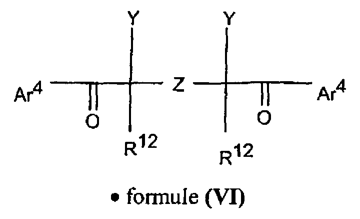 dans laquelle :
   - **R¹²**, identiques ou différents, ont les mêmes significations que dans la formule **(IV),**
   - **Y,** identiques ou différents, représentent X et/ou R⁴,
   - **Z** représente :
      - une liaison directe,
      - un radical divalent alkylène en C₁-C₆, ou un radical phénylène, diphénylène ou phénylène-T-phénylène (T: alkyle linéaire ou ramifié en C₁-C₁₂), ou encore forme, avec les deux substituants R¹² et les deux atomes de carbone porteurs de ces substituants, un noyau de cyclopentane ou de cyclohexane,
      un groupe divalent -O-R¹⁹-O-, -O-SiR¹⁵R¹⁶-O-SiR¹⁵R¹⁶-O-, ou -O-SiR¹⁵R¹⁶-O-,
   - **R²⁰** représente un radical alkylène en C₂-C₈, alcénylène en C₄-C₆ ou xylylène,
   - ou bien encore l'entité : 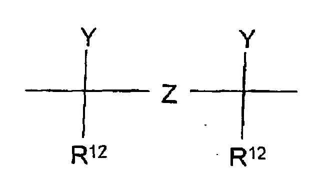 correspond à -O-O-
   - et **Ar⁴** a la même signification que Ar¹ de la formule **(IV)** dans le cas où n = 1.

- famille des thioxanthones de formule **(VII) :** 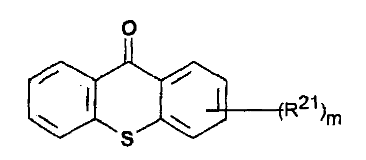
   - m = à 8,
   - **R²¹,** identique(s) ou différent(s) substituants sur le(s) noyau(x) aromatique(s), représentent un radical alkyle linéaire ou ramifié en C1-C12, un radical cycloalkyle en C6-C12, un radical Ar¹, un atome d'halogène, un groupement -OH, -OR¹³ ,-CN, -NO₂, -COOR¹³,-OCOR¹³, -N(R¹³)₂,
   - O-R¹³-(NR¹³)₂ -CHO, -O-phényle, -CF₃, -SR¹³, -S-phényle , -SO₂-phényle , -O-alcényle ou - Si(R¹³)₃.
- famille des xanthènes de formule **(VIII)** : 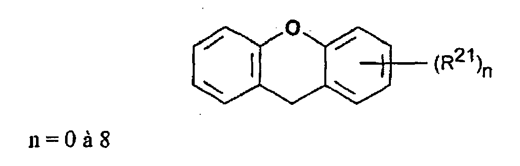
- famille des xanthones de formule **(IX):** 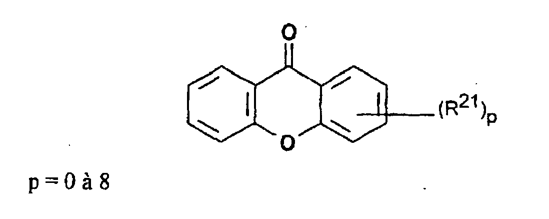
- famille du naphtalène de formule **(X):** 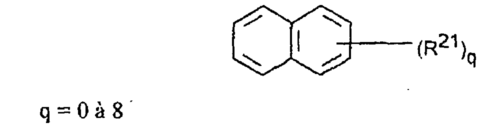
- famille de l'anthracène de formule **(XI) :** 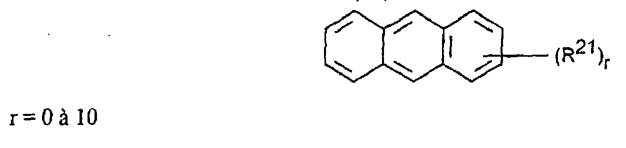
- famille du phénanthrène de formule **(XII) :** 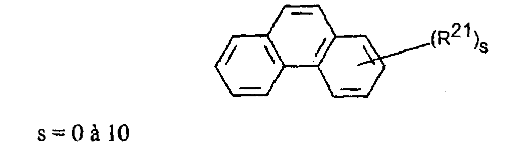
- famille du pyrène de formule **(XIII) :** 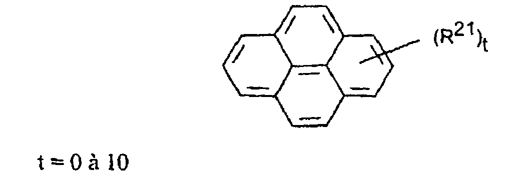
- famille du fluorène de formule **(XIV) :** 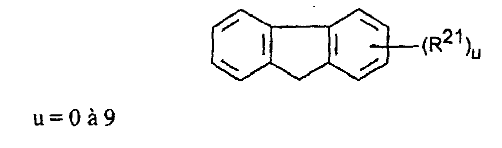
- famille du fluoranthène de formule **(XV) :** 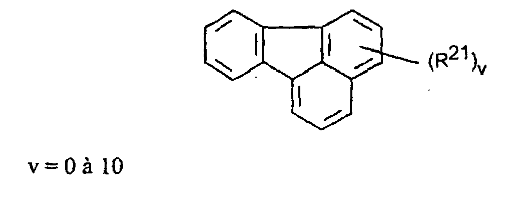
- famille du chrysène de formule **(XVI) :** 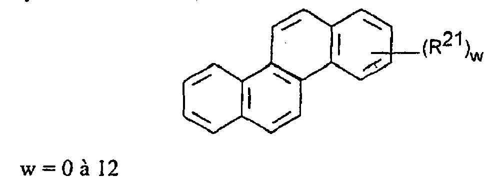
- famille de la fluorène de formule **(XVII) :** 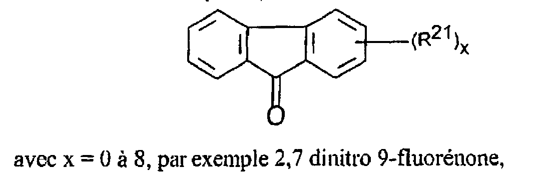
- famille de la chromone de formule **(XVIII) :** 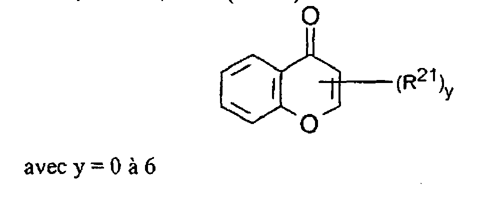
- famille de l'éosine de formule **(XIX) :** 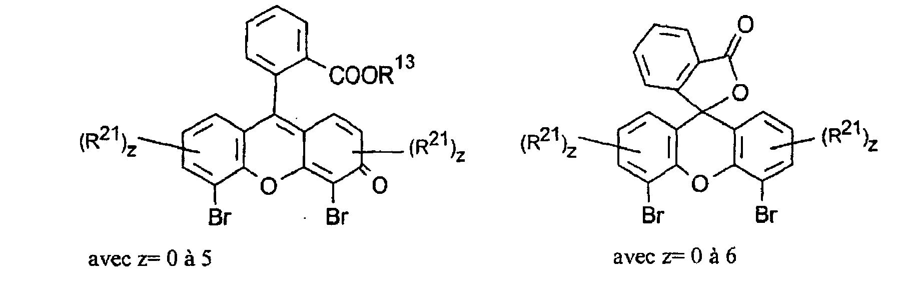
- famille de l'érythrosine de formule **(XX) :** 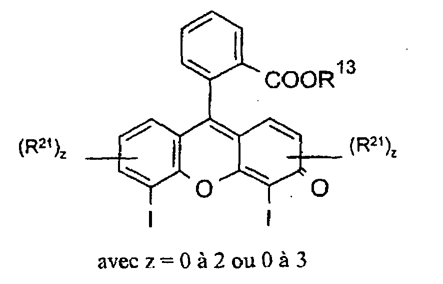
- famille de l'érythrosine de formule **(XXI) :** 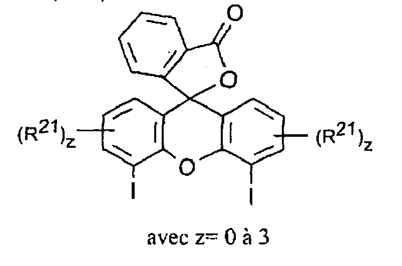
- famille des biscoumarines de formule **(XXII) :** 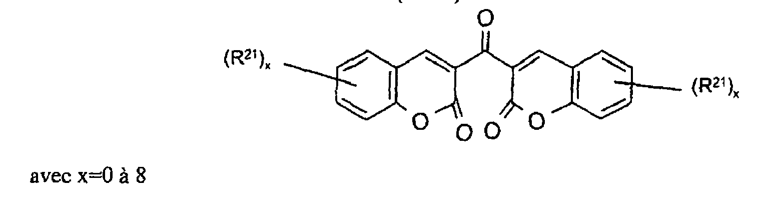
- famille des thioxanthones de formule **(XXIII) :** 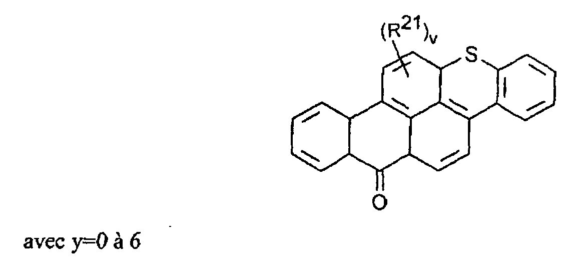
- famille des thioxanthones de formule **(XXIV) :**

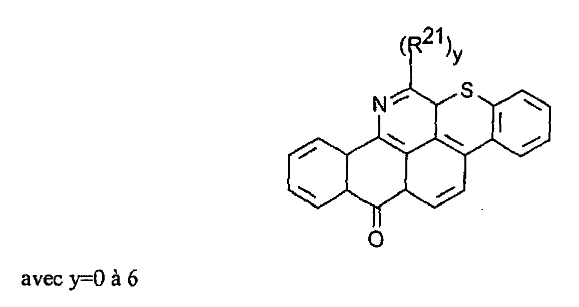

Pour les formules **(VIII)** à **(XXIII),** le groupement **R²¹** a la même définition que pour la molécule **(VII).**

D'autres sensibilisateurs sont utilisables. Notamment, on peut utiliser les photosensibilisateurs décrits dans les documents US 4,939,069; US 4,278,751; US 4,147,552 ainsi que le groupe constitué par les composés de la famille des coumarines, dicétones-conjuguées, fluorones, aminocétones, cétones para-aminostyrylique ainsi que leurs mélanges.

Selon un mode préférentiel, le photosensibilisateur **(E)** est choisi parmi le groupe constitué par les composés de la classe des anthracènes, thioxanthones, camphorquinones, et phénanthrènequinone ainsi que leurs mélanges.

Selon un autre mode préférentiel, la composition dentaire comprend comme photosensibilisateur **(E)** un sel d'une thioxanthone substituée par au moins un groupement comprenant une fonction ammonium. L'utilisation de ce type de photosensibilateur a pour avantage d'éviter les colorations parasitaires lorsque la composition dentaire est réticulée pour la réalisation de prothèse dentaire.

Selon une variante de l'invention, l'anion asssocié du sel de la thioxanthone substituée par au moins un groupement comprenant une fonction ammonium est choisi parmi les anions suivants :
- un halogénure, BF₄⁻, PF₆⁻; SbF₆⁻; l'anion **(III)** de formule [BXₐ R_{b}]⁻ tel que défini ci-dessus, R_{f}SO₃⁻ ; (R_{f}SO₂)₃C⁻ ou (R_{f}SO₂)₂N⁻ avec R_{f} étant un radical alkyle linéaire ou ramifié, substitué par au moins un atome d'halogène, préférentiellement un atome de fluor.

Selon une autre variante de l'invention, le photosensibilisateur **(E),** éventuellement en association avec au moins une camphorquinone, une phénanthrènequinone et/ou un anthracène substitué, a pour formule: 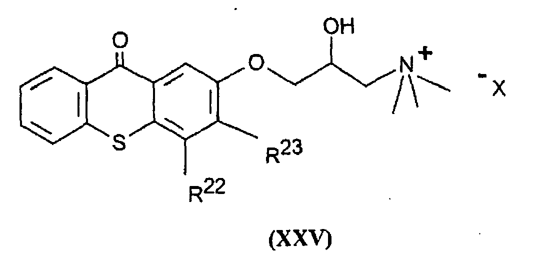 formule dans laquelle :
- **R²²** et **R²³** sont identiques ou différents et représentent un hydrogène ou un radical alkyle en C1-C10, éventuellement substitué, de préférence R22=R23= méthyle,
- (X⁻) étant une entité anionique, de préférence un halogénure; BF₄⁻, PF₆⁻; SbF₆⁻; l'anion **(III)** de formule [BXₐ R_{b}]⁻ défini comme ci-dessus, R_{f}SO₃⁻ ; (P_{f}SO₂)₃C⁻ ou (R_{f}SO₂)₂N⁻, avec R_{f} étant un radical alkyle linéaire ou ramifié, substitué par au moins un atome d'halogène, préférentiellement un atome de fluor,
   et encore plus préférentiellement (X⁻) est choisi parmi les borates de formules suivantes : [B(C₆H₃(CF₃)₂)₄]⁻ et [B(C₆F₅)₄]⁻.

Selon un mode préféré, le photosensibilisateur **(E),** éventuellement en association avec au moins une camphorquinone, une phénanthrènequinone et/ou un anthracène substitué a pour formule:

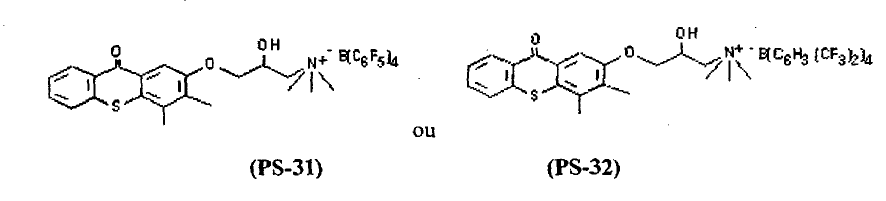

Dans le cadre de la présente invention, les photosensibilisateurs ont une absorption résiduelle de la lumière U.V. comprise entre 200 et 500 nm, de préférence 400 à 500 nm pour les préparations de prothèses dentaires. Pour la restauration dentaire, on préférera un photosensibilisateur ayant une absorption résiduelle de la lumière U.V. au-delà de 400 nm.

Selon une variante préférée, les photosensibilisateurs seront choisis parmi ceux des familles **(VII), (X), (XI), (XIII), (XXIII), (XXIV) et (XXV).** Selon un autre mode de réalisation on choisit le phosensibilisateur parmi le groupe constitué par les composés suivants :
- **(PS-30):** chlorure de 3-(3,4diméthyl-9-oxo-9thioxanthènen-2-yloxy)-2-hydroxy-propyl)-triméthylammonium ;
- **(PS-31):** tétrakis(pentafluorophényl)borate de 3-(3,4diméthyl-9-oxo-9-thioxan-thènen-2-yloxy)-2-hydroxypropyl)-triméthylammonium ;
- **(PS-32):** tétrakis(bis(trifluoromethyl)phényl)borate de 3-(3,4diméthyl-9-oxo-9-thioxanthènen-2yloxy)2-hydroxypropyl)-triméthylammonium;
- **(PS-33):** phénanthrènequinone ;
- **(PS-34):** camphorquinone ;
- **(PS-35) :** Acénaphtènequinone ;
- **(PS-36)** : dibenzoylpéroxyde;
- **(PS-37) :** 2-ethyl-9,10-diméthoxyanthracéne ;
- **(PS-38) :** 9,10-diéthoxyanthracène ;
- **(PS-39) :** 9,10-dibutoxyanthracène;
- **(PS-40) :** 9-hydroxyméthylanthracène ;
- **(PS-41) :** 2-diméthylaminothioxanthoue ;
- **(PS-42) :** 3-éthylcarboxy-7-méthoxythioxanthone ;
- **(PS-43) :** 1-phényl-thio-4-propoxythioxanthone ;
- **(PS-44)** :2-méthoxythioxanthone ;
- **(PS-45) :** 2-(N,Ndiethylaminopropoxy)thioxanthone ;
- **(PS-46) :** 2-isopropylthioxanthone ;
- **(PS-47) :** 1-chloro-4-propoxythioxanthone;
- **(PS-48) :** 4-isopropylthioxanthone ; 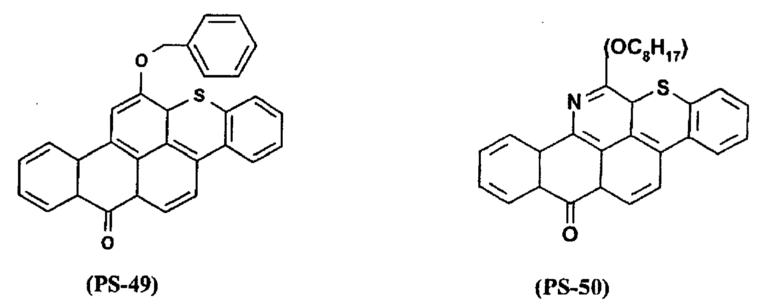
- et leurs mélanges.

Dans le cadre de l'invention, le matériau obtenu après réticulation présente une stabilité au stockage nettement améliorée. Lorsque l'on met en oeuvre les thioxanthones de la famille décrite par la formule **(XXV),** on constate un avantage supplémentaire à utiliser les thioxanthones **(PS-31)** ou **(PS-32),** éventuellement en association avec au moins une camphorquinone, une phénanthrènequinone et/ou un anthracène substitué, qui est l'absence de coloration résiduelle.

Outre les charges de renforcement, des pigments peuvent être utilisés pour teinter la composition dentaire selon l'utilisation envisagée et les groupes ethniques.

Par exemple, on utilise des pigments rouges en présence de microfibres pour les compositions dentaires utilisées pour la préparation de prothèses dentaires afin de simuler les vaisseaux sanguins.

On emploie aussi des pigments à base d'oxydes métalliques (oxydes de fer et/ou titane et/ou aluminium et/ou zirconium, etc.) pour les compositions dentaires utilisées pour la préparation de matériau de restauration, afin d'obtenir un matériau réticulé de couleur ivoire.

D'autres additifs peuvent être incorporés au sein des compositions dentaires selon l'invention, Par exemple, des biocides, des stabilisants, des agents de flaveur, des plastifiants et des promoteurs d'adhérence. Parmi les additifs envisageables, on utilisera avantageusement des co-réactifs réticulables et/ou polymérisables de type organique. Ces co-réactifs sont liquides à température ambiante ou thermofusibles à température inférieure à 100°C, et chaque co-réactif comprend au moins deux fonctions réactives tels que oxétane-alcoxy, oxétane-hydroxy, oxétane-alcoxysilyle, carboxy-oxétane, oxétane-oxétane, alcénylether-hydroxy, alcénylether-alcoxysilyle, époxy-alcoxy, époxy-alcoxysilyles, dioxolane-dioxolane- alcool, etc. On peut citer par exemple les résines R70 et R71.

Les compositions dentaires selon l'invention peuvent être utilisées pour de nombreuses applications dentaires, et en particulier dans le domaine des prothèses dentaires, dans le domaine de la restauration dentaire et dans le domaine des dents provisoires.

La composition dentaire selon l'invention se présente de préférence sous la forme d'un seul produit contenant les différents composants ("*monocomposant*") ce qui facilite sa mise en oeuvre, notamment dans le domaine des prothèses dentaires. Eventuellement, la stabilité de ce produit peut être assurée par des dérivés organiques à fonctions amines selon l'enseignement du document WO 98/07798.

Dans le domaine des prothèses dentaires, le produit sous la forme "monocomposant" peut être déposé à l'aide d'une seringue directement sur le modèle en plâtre ou dans une clé. Puis, il est polymérisé (polymérisation par couches successives possibles) à l'aide d'une lampe UV (spectre lumière visible 400 à 500 nm).

En général, il est possible de réaliser en 10 à 15 min une prothèse dentaire durable et esthétique.

Il est à noter que les produits obtenus à partir de la composition dentaire selon l'invention sont non poreux. Ainsi, après un éventuel polissage à l'aide d'une brosse feutre par exemple, la surface des prothèses dentaires obtenues est lisse et brillante et donc ne nécessite pas d'utilisation de vernis.

Les applications dans le domaine des prothèses dentaires sont essentiellement celles de la prothèse adjointe, que l'on peut diviser en deux types :
- prothèse totale en cas de patient complètement édenté;
- prothèse partielle due à l'absence de plusieurs dents se traduisant par soit une prothèse provisoire, soit un appareil squeletté.

Dans le domaine de la restauration dentaire, la composition dentaire selon l'invention peut être utilisée en tant que matériau d'obturation des dents antérieures et postérieures en différentes teintes (par exemple, teintes "VITA"), rapide et facile à mettre en oeuvre.

La composition dentaire étant non toxique et polymérisable en couches épaisses, il n'est pas indispensable de polymériser le matériau en couches successives. En général, une seule injection de la composition dentaire est suffisante.

Les préparations pour prothèses dentaires et pour matériaux de restauration sont effectuées selon les techniques usuelles du métier.

Dans le cas d'application de la composition dentaire à une dent, soit la dent peut être prétraitée avec agent mordançant puis par un primaire d'accrochage qui peut être lui même photoréticulable ou soit la composition dentaire peut être préparée en mélange avec un primaire d'accrochage avant son utilisation.

L'invention concerne aussi un procédé **(I)** pour traiter une charge dentaire **(B)** comprenant les étapes suivantes :
a) on mélange en milieu solvant la charge dentaire **(B)** et au moins un agent de couplage organosilicié **(F)** tel que défini selon la revendication 1,
b) on élimine le solvant par filtration pour obtenir une charge dentaire intermédiaire **(B-1).**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini selon la revendication 1 et en présence d'au moins un dispersant **(H)** tel que défini selon la revendication 1, 2 ou 3,
e) on élimine le solvant par filtration pour obtenir une charge dentaire intermédiaire **(B-3),** et
f) la charge dentaire intermédiaire **(B-3)** subie un traitement thermique de manière à terminer la réaction entre la charge dentaire intermédiaire **(B-3)** et le composé **(G)** et ainsi d'obtenir une charge dentaire **(B)** traitée.

Selon un autre mode de réalisation, l'invention concerne un procédé **(II)** pour traiter une charge dentaire **(B)** comprenant les étapes suivantes :
a) on mélange en milieu solvant la charge dentaire **(B)** et au moins un agent de couplage organosilicié **(F)** tel que défini selon la revendication 1,
b) on élimine le solvant par filtration pour obtenir une charge dentaire intermédiaire **(B-1),**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini selon la revendication 1,
e) on élimine le solvant par filtration pour obtenir une charge dentaire intermédiaire **(B-3),** et
f) la charge dentaire intermédiaire **(B-3)** subie un traitement thermique de manière à termine la réaction entre la charge dentaire intermédiaire **(B-3)** et le composé **(G)** et ainsi d'obtenir une charge intermédiaire **(B-5),**
g) la charge dentaire intermédiaire **(B-5)** est ensuite mélangée en milieu solvant avec au moins un composé **(G)** tel que défini selon la revendication 1 et en présence d'au moins un dispersant (H) tel que défini selon la revendication 1, 2 ou 3,
h) on élimine le solvant par filtration pour obtenir une charge dentaire intermédiaire **(B-6),** et
i) la charge dentaire intermédiaire **(B-6)** subie un traitement thermique de manière à terminer la réaction entre la charge dentaire intermédiaire **(B-6)** et le composé **(G)** et ainsi d'obtenir une charge dentaire **(B)** traitée.

Selon un mode préférentiel, le traitement thermique des étapes c) et f) des procédés (I) et (II) s'effectuent par un chauffage à une température inférieure ou égale à 200°C, de préférence inférieure ou égale à 160°C et encore plus préférentiellement comprise entre 100 et 165°C.

L'agent de couplage organosilicié **(F),** le composé **(G), le dispersant (H)** et la charge de renforcement dentaire **(B)** sont tels que définis dans la composition dentaire selon l'invention.

L'invention concerne aussi une charge de renforcement susceptible d'être obtenu par le procédé selon l'invention ainsi que son utilisation dans des compositions dentaires.

Un autre objet de l'invention concerne l'utilisation d'une composition dentaire selon l'invention pour la réalisation de prothèses dentaires ou pour la restauration dentaire

Un autre objet de l'invention concerne une prothèse dentaire susceptible d'être obtenue par réticulation d'une composition selon l'invention.

Un dernier objet de l'invention concerne un maatériau de restauration dentaire susceptible d'être obtenu par réticulation d'une composition selon l'invention.

Les Exemples suivants sont donnés à titre illustratif. Ils permettent notamment de mieux comprendre l'invention et de faire ressortir certains de ses avantages et d'illustrer quelques unes de ses variantes de réalisation.

### EXEMPLES

### Structures:

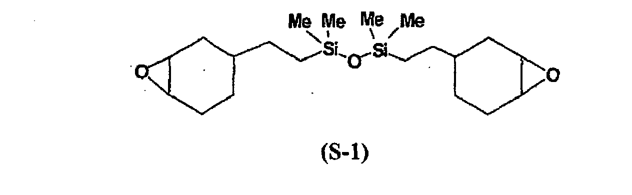

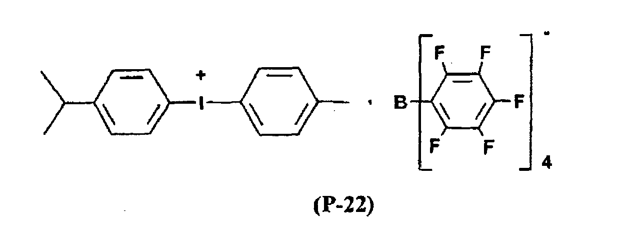

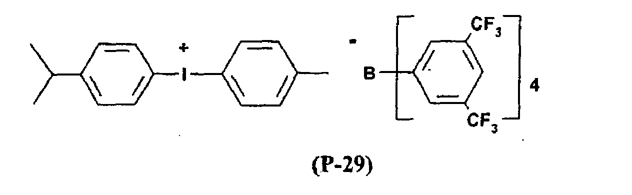

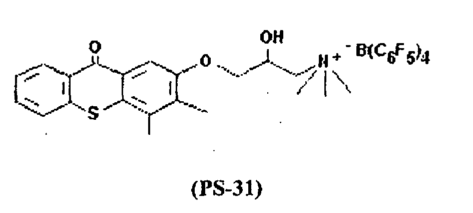

- Composé **(S-96)** commercialisée par la société Dow-Chemical sous la référence UVR-6105 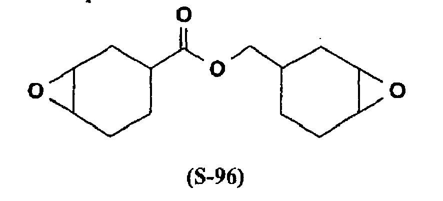
- Charge **(B-i) :** verre de type quartz (granulométrie = 1.5 µm, SiO₂ 56% ;SrO 14% ;B₂O₃ 14% ; Al₂O₃ 14% ; F 2% ) commercialisé par la société Schott sous la référence G018-163
   Charge **(B-ii) :** verre de type quartz (granulométrie = 1.5 µm, SiO₂ 55% ;BaO 25% ; B₂O₃ 10% ; Al₂O₃ 10%) commercialisé par la société Schott sous la référence GM27-884.
   Dispersant **(H) :** Disperbyk-164^{®} (commercialisé par la société Byk).
   Dispersant **(C) :** Disperbyk-164^{®} (commercialisé par la société Byk).

### Exemple 1-1 et 1-2 Traitement d'une charge dentaire selon l'invention :

### Exemple 1-1 :

a) Dans un flacon on verse 200 g de charge **(B-i),** on y ajoute 200 g d'acétone puis 7,3 g de silane glycidyloxypropyltriméthoxysilane ou GLYMO. On mélange pendant deux heures sous agitation à hélice à température ambiante et sous azote. On verse l'ensemble dans un Buchner équipé d'un filtre (0,22 microns). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge intermédiaire **(B-2).**
b) Dans un flacon on verse 180 g de la charge **(B-2),** on y ajoute 180 g d'acétone puis 31,5 g du composé **(S-1)** à fonction oxirane (pour l'exemple 1-2 seulement 15,8g du composé **(S-1)** à fonction oxirane et on ajoute aussi 15,8 g de composé **(S-96))** et 0,85 g d'une solution à 60 % du dispersant **(H)** dans un solvant. On mélange pendant deux heures sous agitation à hélice à température ambiante et sous azote. On verse l'ensemble dans un Buchner équipé d'un filtre (0,22 micron). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge dentaire (B-i) traitée selon l'invention.

### Exemple 2 Traitement d'une charge dentaire selon l'invention :

a) Dans un flacon on verse 200 g de charge (B-i), on y ajoute 200 g d'acétone puis 7,3 g de silane glycidyloxypropyltriméthoxysilane ou GLYMO. On mélange pendant deux heures sous agitation à hélice à température ambiante et sous azote. On verse l'ensemble dans un Buchner équipé d'un filtre (0,22 micron). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge intermédiaire **(B-2).**
b) Dans un flacon on verse 180 g de la charge **(B-2),** on y ajoute 180 g d'acétone puis 15,8 g du composé **(S-1)** à fonction oxirane et 15,8 g de composé **(S-96).** On mélange pendant deux heures sous agitation à hélice à température ambiante et sous azote. On verse l'ensemble dans un Buchner équipé d'un filtre (0,22 microns). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge dentaire intermédiaire (B-5).
c) Dans un flacon on verse 160 g de la charge (B-5), on y ajoute 160 g d'acétone puis 28,1 g du composé **(S-1)** à fonction oxirane et 0,75 g d'une solution à 60 % du dispersant **(H)** dans un solvant. On mélange pendant deux heures sous agitation à hélice à température ambiante et sous azote. On verse l'ensemble dans un Buchner équipé d'un filtre (0,22 microns). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge dentaire (B-i) traitée selon l'invention.

**Exemples 3 (Comparatif) - Traitement d'une charge selon la demande internationale** WO-A-2005121200**:**
a) Dans un flacon on verse 200 g de charge **(B-i),** on y ajoute 200 g d'acétone puis 7,3 g de silane glycidyloxypropyltriméthoxysilane ou GLYMO. On mélange pendant deux heures sous agitation à hélice à température ambiante et sous azote. On verse l'ensemble dans un Buchner équipé d'un filtre (,22 microns). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge intermédiaire (B-2).
b) Dans un flacon on verse 180 g de la charge **(B-2),** on y ajoute 180 g d'acétone puis 15,8 g du composé **(S-1)** à fonction oxirane et 15,8 g de composé **(S-96).** On mélange pendant deux heures sous agitation à hélice à température ambiante et sous azote. On verse l'ensemble dans un Buchner équipé d'un filtre (0,22 microns). On récupère la charge dentaire qui est ensuite broyée à l'aide mortier et d'un pilon puis tamisée sur une toile de 200 microns. La charge dentaire est ensuite séchée à l'étuve pendant 16h à 110°C. A la sortie de l'étuve, la charge est refroidie à température ambiante pendant 30 minutes. On obtient une charge dentaire traitée selon la demande internationale WO-A-2005121200.

### Exemple 4 : Formulations des compositions dentaires :

On charge dans un flacon 0,09g du photosensibilisateur de formule **(PS-31),** on ajoute 0,13g du du photosensibilisateur 9,10-dibutoxyanthracène **(PS-39),** 0,13g du photosensibilisateur camphorquinone **(PS-34),** 3,87g de photoamorceur **(P-22),** 195,1g de résine siloxane à fonction oxirane **(S-1)** enfin on ajoute 0,58g d'une solution à 60 % de dispersant **(C)** dans un solvant. Cette formulation est agitée à l'aide d'un barreau magnétique pendant environ 24 heures.

On charge dans un mortier 7,75 g de la formulation préparée ci-dessus et on ajoute 17,25g d'une charge dentaire :
- pour la Formulation 1 (Comparatif): charge = Charge **(B-i)** non traitée,
- pour la Formulation 2 (Invention): charge = Charge **(B-i)** traitée selon l'Exemple 1-1,
- pour la Formulation 3 (Invention): charge = Charge **(B-i)** traitée selon l'Exemple 1-2,
- pour la Formulation 4 (Invention): charge = Charge **(B-i)** traitée selon l'Exemple 2,
- pour la Formulation 5 (Comparatif): charge = Charge **(B-ii)** traitée selon l'Exemple 3 ; et on malaxe le mélange à l'aide d'un mortier et d'un pilon.

Puis on suit l'évolution du taux d'époxy au cours du temps des formulations avant réticulation qui sont placées dans une étuve à 40°C (vieillissement accéléré et simulation d'un stockage longue durée). Les résultats sont consignés dans le Tableau I suivant :

**Tableau I : Taux époxy (mEE = mEquivalent Epoxy) après vieillissement de x jours à 40°C**

| **Nombre de jours (x) à 40°C** | **Formulation 1 Comparatif** | **Formulation 5 Comparatif** | **Formulation 4 Invention** | **Formulation 3 Invention** | **Fomulation 2 Invention** |
|---|---|---|---|---|---|
| **0** | **138** | **130** | **143** | **171** | **154** |
| **1** | **69** | **126** | **142** | **170** | **153** |
| **4** | | **111** | **138** | **168** | **150** |
| **6** | | **102** | **136** | **167** | **148** |
| **12** | | | **129** | **162** | **143** |
| **20** | | | **121** | **157** | **136** |
| **30** | | | **110** | **150** | **128** |
| **35** | | | **104** | **146** | **123** |
| **40** | | | **99** | **142** | **119** |
| **50** | | | | **135** | |

On remarque que:
- la limite acceptable d'utilisation est d'environ 100 mEE, en dessous de ce seuil la formulation se présente sous une forme durcie et n'est plus utilisable en l'état,
- pour une formulation à base d'une charge non traitée (Formulation 1), le taux d'époxy mesuré chute de près de 50 % après 24 h de vieillissement accélérée à 40°C. Ceci indique que la formulation n'est pas stable car les fonctions époxy ont réagit (durcissement néfaste de la formulation) ;
- pour une formulation à base d'une charge traitée selon la demande internationale WO-A-2005121200 (Formulation 5), le taux d'époxy mesuré chute de près de 30 % après 6 jours de vieillissement accélérée à 40°C ; et
- pour les formulations selon l'invention (Formulations 2, 3 et 4), les taux d'époxy sont plus stable ce qui évite un durcissement prématuré des formulations lors d'un stockage de longue durée.

## Revendications

1. Composition dentaire comprenant :
(1) au moins un composé **(A)** réactif par voie cationique.
(2) au moins une charge dentaire **(B),**
(3) éventuellement au moins une charge dentaire **(B'),**
(4) éventuellement au moins un dispersant **(C)** comprenant au moins un polymère ou copolymère organique ;
(5) au moins un photoamorceur cationique **(D);** et
(6) éventuellement au moins un photosensibilisateur **(E),**
ladite composition étant **caractérisée en ce que** la charge dentaire **(B)** est préalablement traitée en milieu solvant par mise en contact avec les ingrédients (a), (b) et (c) suivants:
(a) au moins un composé **(G)** qui est:
- un monomère, oligomère ou polymère organique, ou
- un monomère, oligomère ou polymère organosiloxane, ledit composé **(G)** comprenant au moins une fonction réactive **(frC)** oxirane, oxétane, alcényle éther et/ou carbonate,
(b) au moins un agent de couplage organosilicié **(F)** comprenant au moins une fonction réactive **(frA)** directement liée à un atome de silicium susceptible de réagir avec la charge dentaire **(B)** et au moins une fonction réactive **(frB)** non directement liée à un atome de silicium susceptible de réagir avec une fonction réactive **(frC)** du composé **(G),** et
(c) au moins un dispersant **(H)** permettant de maintenir la charge dentaire **(B)** sous une forme non agglomérée pendant et/ou après le traitement en milieu solvant.

2. Composition dentaire selon la revendication 1 dans laquelle le dispersant **(H)** est un polymère ou copolymère organique ou un monodiester d'acide carboxylique.

3. Composition dentaire selon la revendication 1 dans laquelle le dispersant **(H)** est choisi parmi le groupe constitué par:
- les polymères acryliques éventuellement salifiés par un alkylammonium, les polyesters, les polyéthers, les polyuréthannes, les polyuréthannes modifiés, les polyol-polyacrylates,
- leurs copolymères,
- les monodiesters d'acides carboxyliques,
- les copolymères polyuréthanne/acrylate éventuellement salifiés par un alkylammonium, et
- leurs mélanges.

4. Composition dentaire selon l'une quelconque des revendications 1 à 3 dans laquelle au moins une charge dentaire **(B)** est traitée par un procédé **(I)** comprenant les étapes suivantes :
a) on mélange en milieu solvant la charge dentaire **(B)** et au moins un agent de couplage organosilicié **(F)** tel que défini selon la revendication 1,
b) on élimine le solvant par filtration pour obtenir une charge dentaire intermédiaire **(B-1),**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini selon la revendication 1 et en présence d'au moins un dispersant **(H)** tel que défini selon la revendication 1, 2 ou 3,
e) on élimine le solvant par filtration pour obtenir une charge dentaire intermédiaire **(B-3),** et
f) la charge dentaire intermédiaire **(B-3)** subie un traitement thermique de manière à terminer la réaction entre la charge dentaire intermédiaire **(B-3)** et le composé **(G)** et ainsi d'obtenir une charge dentaire **(B)** traitée.

5. Composition dentaire selon l'une quelconque des revendications 1 à 3 dans laquelle au moins une charge dentaire **(B)** est traitée par un procédé **(II)** comprenant les étapes suivantes :
a) on mélange en milieu solvant la charge dentaire **(B)** et au moins un agent de couplage organosilicié **(F)** tel que défini selon la revendication 1,
b) on élimine le solvant par filtration pour obtenir une charge dentaire intermédiaire **(B-1),**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini selon la revendication 1,
e) on élimine le solvant par filtration pour obtenir une charge dentaire intermédiaire **(B-3),** et
f) la charge dentaire intermédiaire **(B-3)** subie un traitement thermique de manière à terminer la réaction entre la charge dentaire intermédiaire **(B-3)** et le composé **(G)** et ainsi d'obtenir une charge intermédiaire **(B-5),**
g) la charge dentaire intermédiaire **(B-5)** est ensuite mélangée en milieu solvant avec au moins un composé **(G)** tel que défini selon la revendication 1 et en présence d'au moins un dispersant **(H)** tel que défini selon la revendication 1, 2 ou 3,
h) on élimine le solvant par filtration pour obtenir une charge dentaire intermédiaire **(B-6),** et
i) la charge dentaire intermédiaire **(B-6)** subie un traitement thermique de manière à terminer la réaction entre la charge dentaire intermédiaire **(B-6)** et le composé **(G)** et ainsi d'obtenir une charge dentaire t **(B)** traitée.

6. Composition dentaire selon la revendication 4 ou 5 dans laquelle les traitements thermiques des étapes c), f) et i) des procédés **(I)** et **(II)** s'effectuent par un chauffage à une température inférieure ou égale à 200°C, de préférence inférieure ou égale à 165°C et encore plus préférentiellement comprise entre 100 et 165°C.

7. Composition dentaire selon la revendication 1 dans laquelle :
- la fonction réactive **(frA)** directement liée à un atome de silicium de l'agent de couplage organosilicié **(F)** est une fonction alcoxy, énoxy ou hydroxy;
- la fonction réactive **(frB)** non directement liée à un atome de silicium de l'agent de couplage organosilicié **(F)** est une fonction oxirane, oxétane, hydroxy, acide, anhydride d'acide carboxylique ou diol; et
- la fonction réactive **(frC)** du composé **(G)** est une fonction oxirane, oxétane, alcényle éther ou carbonate.

8. Composition dentaire selon l'une quelconque des revendications précédentes dans laquelle l'agent de couplage organosilicié **(F)** est choisi parmi le groupe constitué par les composés suivants: le glycidyloxypropyltriméthoxysilane, le produit d'hydrolyse du glycidyloxypropyltriméthoxysilane; le glycidyloxypropyltriéthoxysilane, le produit d'hydrolyse en milieu acide du glycidyloxypropyltriéthoxysilane ; le glycidyloxypropyldiméthoxyméthylsilane ou le produit d'hydrolyse, le béta-(3,4-époxycyclohexyl)éthyltriéthoxysilane ou le produit d'hydrolyse et le béta-(3,4-époxycyclohexyl)éthyltriméthoxysilane ou le produit d'hydrolyse.

9. Composition dentaire selon l'une quelconque des revendications précédentes dans laquelle le composé **(G)** est un oligomère silicone **(G-1)** ou un polymère silicone **(G-2)** comprenant au moins une fonction réactive **(frC)** oxirane, oxétane, alcényle éther et/ou carbonate.

10. Composition dentaire selon la revendication 9 dans laquelle l'oligomère silicone **(G-1)** ou le polymère silicone **(G-2)** comprennent :
a) au moins un motif de formule: 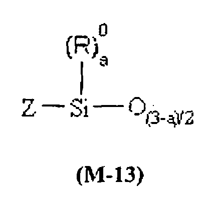 formule dans laquelle :
- a = 0, 1 ou 2,
- R⁰, identique ou différent, représente un radical alkyle, cycloalkyle, aryle, vinyle, hydrogéno, alcoxy, de préférence un alkyle inférieur en C₁-C₆,
- Z, identique ou différent, est un substituant organique comportant au moins une fonction oxirane, alcénylether, oxétane et/ou carbonate, et
b) au moins deux atomes de silicium.

11. Composition dentaire selon la revendication 10 dans laquelle l'oligomère silicone **(G-1)** ou le polymère silicone **(G-2)** sont choisis parmi le groupe constitué par les composés de formules **(S-1)** à **(S-15)** suivantes:
**a)** 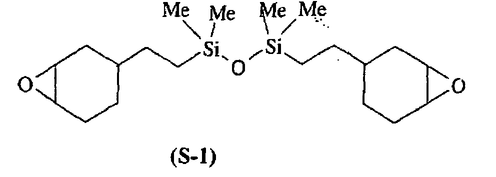
**b)** 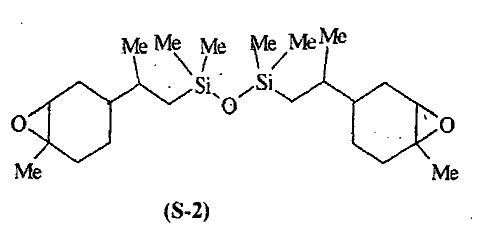
**c)** 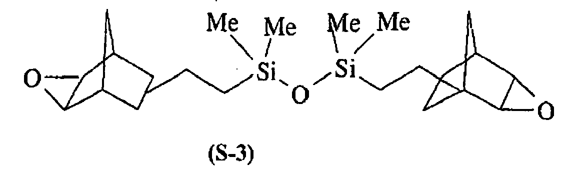
**d)** 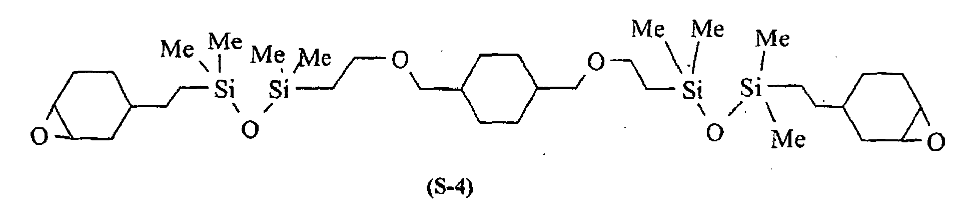
**e)** 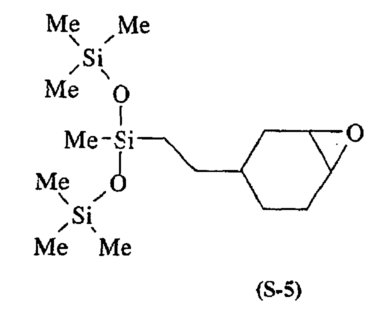
**f)** 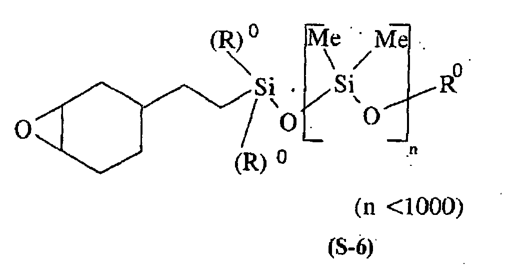
**g)** 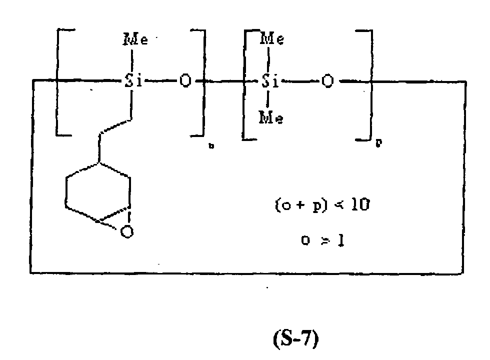
**h)** 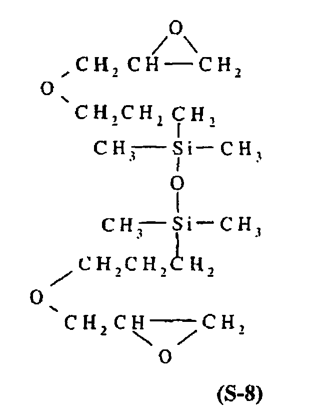
**i)** 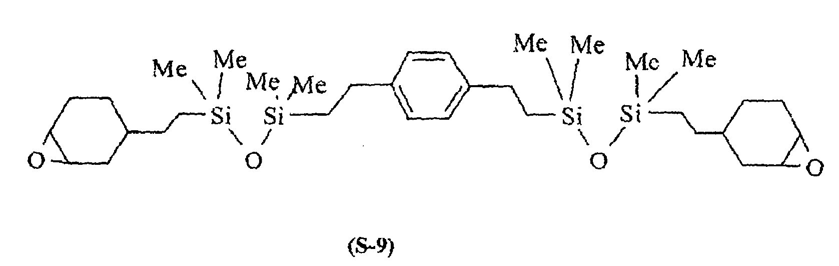
**j)** 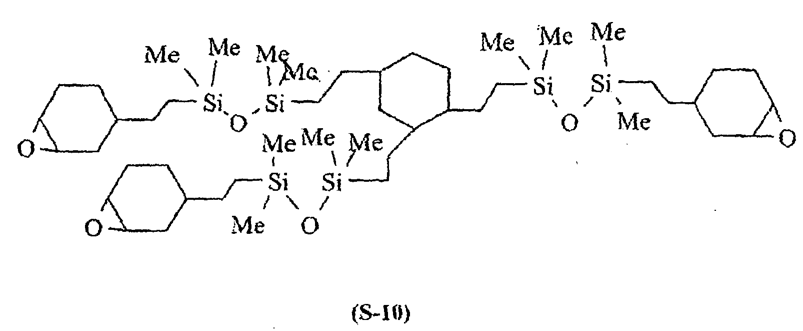
**k)** 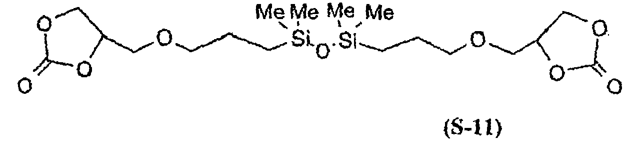
**l)** 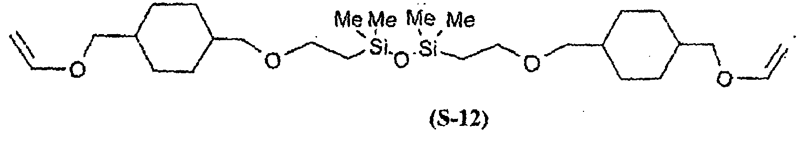
**m)** 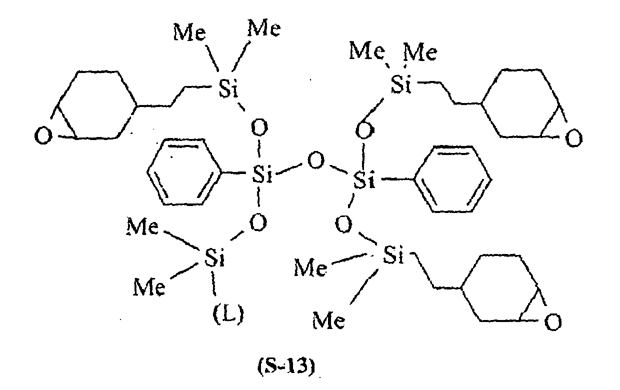 L= H ; OH ; Me ; phényle; alkyle en C₁-C₁₂ ; cycloalkyle en C₄-C₆ ou les groupements suivants: 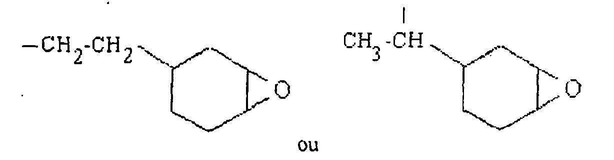
**n)** 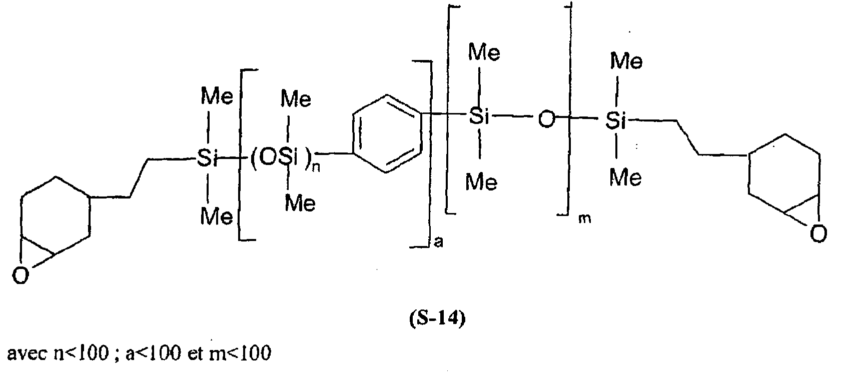
**o)** 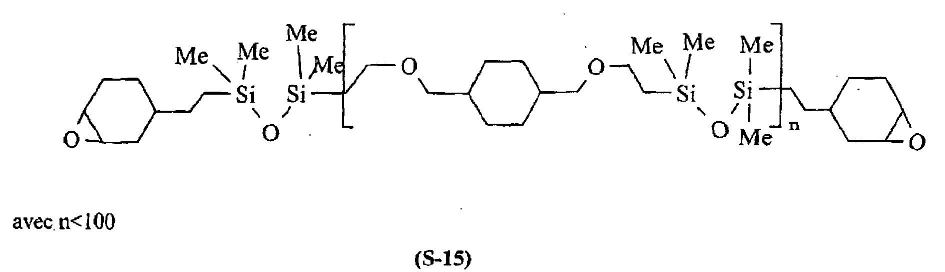 formules dans lesquelles R^{o} ou R₀ , identiques ou différents, représente un radical alkyle, cycloalkyle, aryle, de préférence un alkyle inférieure en C₁-C₆.

12. Composition selon la revendication 1, **caractérisée en ce que** le photoamorceur cationique (D) est choisi parmi le groupe constitué par les composés suivants :
**(P-16)** : [(C₈H₁₇)-O- C₆H₄-I- C₆H₅))⁺, [B(C₆F₅)₄]⁻;
**(P-17)** : [C₁₂H₂₅- C₆H₄-I-C₆H₅]⁻, [B(C₆F₅)₄]⁻;
**(P-18) :** [[(C₈H₁₇-O- C₆H₄)₂I]⁺, [B(C₆F₅)₄]⁻;
**(P-19) :** [(C₈H₁₇)-O- C₆H₄-I-C₆H₅)]⁻, [B(C₆F₅)₄]⁻;
**(P-20) :** [(C₆H₅)₂S- C₆H₄-O-C₈H₁₇]⁻, [B(C₆H₄CF₃)₄]⁻;
**(P-21) :** [(C₁₂H₂₅- C₆H₄)₂I]⁺, [B(C₆F₅)⁴]⁻;
**(P-22) :** [CH₃- C₆H₄-I- C₆H₄-CH(CH₃)₂]⁺, [B(C₆F₅)₄]⁻;
**(P-23) :** (η5 - cyclopentadiènyle) (η6 - toluène) Fe⁻, [B(C₆F₅)₄]⁻;
**(P-24) :** (η5 - cyclopentadiènyle) (η6 - methyl-1-naphtalène) Fe⁻, [B(C₆F₅)⁴]⁻ ;
**(P-25) :** (η5 - cyclopentadiènyle) (η6 cumène) Fe⁺, [B(C₆F₅)₄]⁻;
**(P-26) :** [(C₁₂H₂₅- C₆H₄)₂I]⁺, [B(C₆H₃(CF₃)₂]⁻;
**(P-27) :** [CH₃- C₆H₄-I- C₆H₄-CH₂CH(CH₃)₂]⁺, [B(C₆F₅)₄]⁻ ;
**(P-28) :** [CH₃- C₆H₄-I- C₆H₄-CH₂CH(CH₃)₂]⁺, [B(C₆H₃(CF₃)₂)₄]⁻; et
**(P-29) :** [CH₃- C₆H₄-I- C₆H₄-CH(CH₃)₂]⁻, [B(C₆H₃(CF₃)₂)₄)⁻.

13. Composition dentaire selon la revendication 1, **caractérisée en ce que** le photoamorceur cationique (D) est un sel d'iodonium.

14. Composition dentaire selon la revendication 1 dans laquelle le photosensibilisateur **(E)** comprend dans sa structure un ou plusieurs noyaux aromatiques substitués ou non, ayant une absorption résiduelle de la lumière comprise entre 200 et 500 nm.

15. Composition dentaire selon la revendication 1 dans laquelle le photosensibilisateur **(E)** est choisi parmi le groupe constitué par les composés de la classe des anthracènes, thioxanthones, camphorquinones, et phénanthrènequinone ainsi que leurs mélanges.

16. Composition dentaire selon l'une quelconque des revendications précédentes dans laquelle la charge dentaire **(B)** est un verre minéral ou une silice de combustion.

17. Procédé pour traiter une charge dentaire **(B)** comprenant les étapes suivantes :
a) on mélange en milieu solvant la charge dentaire **(B)** et au moins un agent de couplage organosilicié **(F)** tel que défini selon la revendication 1,
b) on élimine le solvant par filtration pour obtenir une charge dentaire intermédiaire **(B-1),**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini selon la revendication 1 et en présence d'au moins un dispersant **(H)** tel que défini selon la revendication 1, 2 ou 3,
e) on élimine le solvant par filtration pour obtenir une charge dentaire intermédiaire **(B-3),** et
f) la charge dentaire intermédiaire **(B-3)** subie un traitement thermique de manière à terminer la réaction entre la charge dentaire intermédiaire **(B-3)** et le composé **(G)** et ainsi d'obtenir une charge dentaire **(B)** traitée.

18. Procédé pour traiter une charge dentaire **(B)** comprenant les étapes suivantes :
a) on mélange en milieu solvant la charge dentaire **(B)** et au moins un agent de couplage organosilicié **(F)** tel que défini selon la revendication **1,**
b) on élimine le solvant par filtration pour obtenir une charge dentaire intermédiaire **(B-1).**
c) la charge dentaire intermédiaire **(B-1)** subie un traitement thermique de manière à terminer la réaction de couplage entre la charge dentaire intermédiaire **(B-1)** et l'agent de couplage **(F)** et ainsi d'obtenir une charge dentaire intermédiaire **(B-2),**
d) la charge dentaire intermédiaire **(B-2)** est ensuite mélangée en milieu solvant avec au moins un composé **(G)** tel que défini selon la revendication 1,
e) on élimine le solvant par filtration pour obtenir une charge dentaire intermédiaire **(B-3)**, et
f) la charge dentaire intermédiaire **(B-3)** subie un traitement thermique de manière à terminer la réaction entre la charge dentaire intermédiaire **(B-3)** et le composé **(G)** et ainsi d'obtenir une charge intermédiaire **(B-5),**
g) la charge dentaire intermédiaire **(B-5)** est ensuite mélangée en milieu solvant avec au moins un composé **(G)** tel que défini selon la revendication 1 et en présence d'au moins un dispersant **(H)** tel que défini selon la revendication 1, 2 ou 3.
h) on élimine le solvant par filtration pour obtenir une charge dentaire intermédiaire **(B-6),** et
i) la charge dentaire intermédiaire **(B-6)** subie un traitement thermique de manière à terminer la réaction entre la charge dentaire intermédiaire **(B-6)** et le composé **(G)** et ainsi d'obtenir une charge dentaire **(B)** traitée.

19. Charge dentaire susceptible d'être obtenue par le procédé selon la revendication 17 ou 18.

20. Utilisation d'une charge dentaire selon la revendication 19 dans des compositions dentaires.

21. Utilisation d'une composition dentaire selon l'une quelconque des revendications 1 à 16, pour la réalisation de prothèses dentaires ou pour la restauration dentaire.

22. Prothèse dentaire susceptible d'être obtenue par réticulation d'une composition selon l'une quelconque des revendications 1 à 16.

23. Matériau de restauration dentaire susceptible d'être obtenu par réticulation d'une composition selon l'une quelconque des revendications 1 à 16.

## Claims

1. Dental composition comprising:
(1) at least one cationically reactive compound **(A),**
(2) at least one dental filler **(B),**
(3) optionally at least one dental filler **(B'),**
(4) optionally at least one dispersant **(C),** comprising at least one organic polymer or copolymer;
(5) at least one cationic photoinitiator **(D);** and
(6) optionally at least one photosensitizer **(E),** said composition being **characterized in that** the dental filler **(B)** is pretreated in a solvent medium by contacting it with the following ingredients (a), (b), and (c):
(a) at least one compound **(G)** which is:
- an organic monomer, oligomer or polymer, or
- an organosiloxane monomer, oligomer or polymer, said compound **(G)** comprising at least one reactive oxirane, oxetane, alkenyl ether and/or carbonate function (**frC**),
(b) at least one organosilicon coupling agent **(F)** comprising at least one reactive function **(frA)** bonded directly to a silicon atom capable of reacting with the dental filler **(B),** and at least one reactive function **(frB)** not directly bonded to a silicon atom capable of reacting with a reactive function **(frC)** of the compound (G), and
(c) at least one dispersant **(H)** which maintains the dental filler **(B)** in a nonagglomerated form during and/or after the treatment in a solvent medium.

2. Dental composition according to Claim 1, wherein the dispersant **(H)** is an organic polymer or copolymer or a carboxylic acid monodiester.

3. Dental composition according to Claim 1, wherein the dispersant **(H)** is selected from the group consisting of:
- acrylic polymers optionally in the form of a salt with an alkylammonium, polyesters, polyethers, polyurethanes, modified polyurethanes, and polyacrylate polyols,
- copolymers thereof,
- carboxylic acid monodiesters,
- polyurethane/acrylate copolymers, optionally in the form of a salt with an alkylammonium, and
- mixtures thereof.

4. Dental composition according to any one of Claims 1 to 3, wherein at least one dental filler **(B)** is treated by a process **(I)** comprising the following steps:
a) in a solvent medium the dental filler **(B)** and at least one organosilicon coupling agent **(F)** as defined in Claim 1 are mixed,
b) the solvent is removed by filtration to give an intermediate dental filler (**B-1**),
c) the intermediate dental filler (**B-1**) undergoes a heat treatment to terminate the coupling reaction between the intermediate dental filler (**B-1**) and the coupling agent **(F)** and so to obtain an intermediate dental filler **(B-2),**
d) the intermediate dental filler **(B-2)** is then mixed in a solvent medium with at least one compound **(G)** as defined in Claim 1 and in the presence of at least one dispersant **(H)** as defined in Claim 1, 2 or 3,
e) the solvent is removed by filtration to give an intermediate dental filler **(B-3),** and
f) the intermediate dental filler **(B-3)** undergoes a heat treatment to terminate the reaction between the intermediate dental filler **(B-3)** and the compound. **(G)** and so to obtain a treated dental filler (**B**) .

5. Dental composition according to any one of Claims 1 to 3, wherein at least one dental filler **(B)** is treated by a process **(II)** comprising the following steps:
a) in a solvent medium the dental filler **(B)** and at least one organosilicon coupling agent **(F)** as defined in Claim 1 are mixed,
b) the solvent is removed by filtration to give an intermediate dental filler (**B-1**).
c) the intermediate dental filler (**B-1**) undergoes a heat treatment to terminate the coupling reaction between the intermediate dental filler (**B-1**) and the coupling agent **(F)** and so to obtain an intermediate dental filler (**B-2**),
d) the intermediate dental filler **(B-2)** is then mixed in a solvent medium with at least one compound **(G)** as defined in Claim 1,
e) the solvent is removed by filtration to give an intermediate dental filler **(B-3),**
f) the intermediate dental filler **(B-3)** undergoes a heat treatment to terminate the reaction between the intermediate dental filler **(B-3)** and the compound **(G)** and so to obtain an intermediate filler **(B-5),**
g) the intermediate dental filler **(B-5)** is then mixed in a solvent medium with at least one compound **(G)** as defined in Claim 1 and in the presence of at least one dispersant **(H)** as defined in Claim 1, 2 or 3,
h) the solvent is removed by filtration to give an intermediate dental filler **(B-6),** and
i) the intermediate dental filler **(B-6)** undergoes a heat treatment to terminate the reaction between the intermediate dental filler **(B-6)** and the compound **(G)** and so to obtain a treated dental filler **(B).**

6. Dental composition according to Claim 4 or 5, wherein the heat treatments of steps c), f) and i) of processes (I) and **(II)** take place by heating to a temperature less than or equal to 200°C, preferably less than or equal to 165°C and more preferably between 100 and 165°C.

7. Dental composition according to Claim 1, wherein:
- the reactive function **(frA)** bonded directly to a silicon atom of the organisilicon coupling agent **(F)** is an alkoxy, enoxy or hydroxyl function;
- the reactive function **(frB)** not bonded directly to a silicon atom of the organosilicon coupling agent **(F)** is an oxirane, oxetane, hydroxyl, acid, carboxylic acid anhydride or diol function; and
- the reactive function (**frC**) of the compound **(G)** is an oxirane, oxetane, alkenyl ether or carbonate function.

8. Dental composition according to any one of the preceding claims, wherein the organosilicon coupling agent **(F)** is selected from the group consisting of the following compounds: glycidyloxypropyltrimethoxysilane, the product of hydrolysis of glycidyloxypropyltrimethoxysilane; glycidyloxypropyltriethoxysilane, the product of hydrolysis in acidic medium of glycidyloxypropyltriethoxysilane; glycidyloxypropyldimethoxymethylsilane or the product of hydrolysis, beta-(3,4-epoxycyclohexyl)ethyltriethoxysilane or the product of hydrolysis, and beta-(3,4-epoxycyclohexyl) ethyltrimethoxysilane or the product of hydrolysis.

9. Dental composition according to any one of the preceding claims, wherein the compound **(G)** is a silicone oligomer **(G-1)** or a silicone polymer **(G-2)** comprising at least one reactive oxirane, oxetane, alkenyl ether and/or carbonate function (**frC**).

10. Dental composition according to Claim 9, wherein the silicone oligomer **(G-1)** or the silicone polymer **(G-2)** comprise:
a) at least one unit of formula: 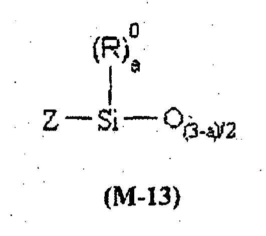 in which formula:
- a = 0, 1 or 2,
- R⁰, identical or different at each occurrence, represents an alkyl, cycloalkyl, aryl, vinyl, hydrogeno or alkoxy radical, preferably a C₁-C₆ lower alkyl,
- Z, identical or different at each occurrence, is an organic substituent containing at least one oxirane, alkenyl ether, oxetane and/or carbonate function, and
b) at least two silicon atoms.

11. Dental composition according to Claim 10, wherein the silicone oligomer (**G-1**) or the silicone polymer **(G-2)** are selected from the group consisting of the compounds of formulae (**S-1**) to (**S-15**) below:
**a)** 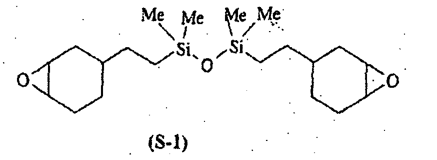
**b)** 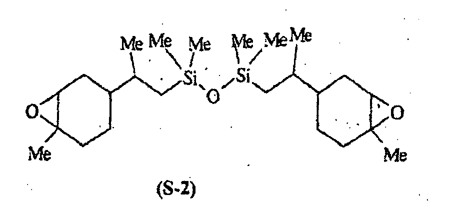
**c)** 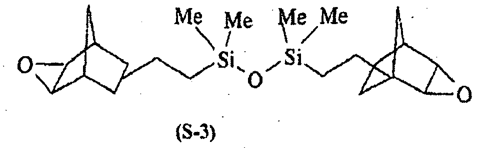
**d)** 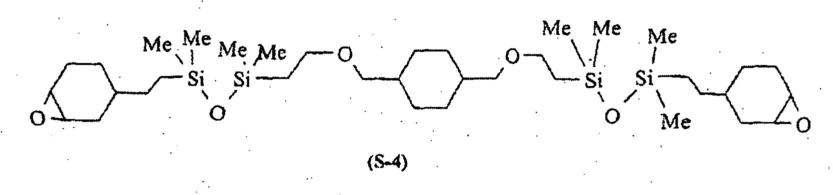
**e**) 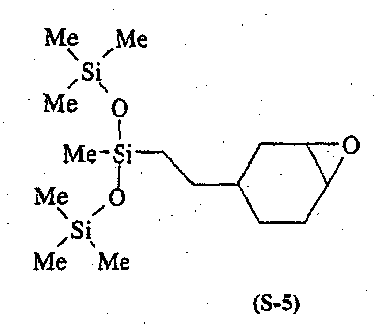
**f**) 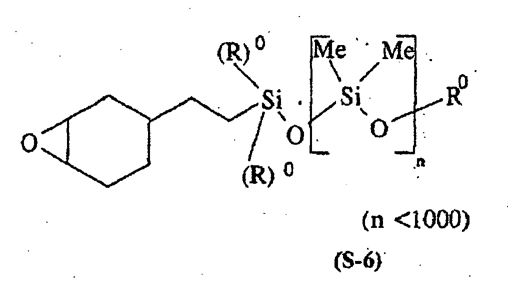
**g)** 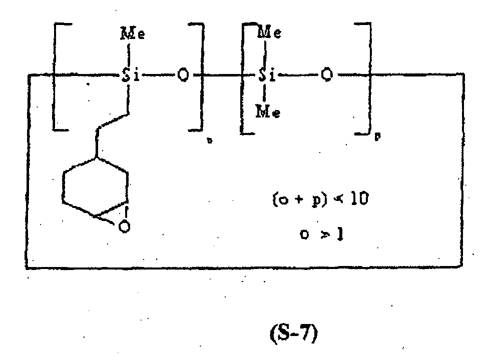
**h)** 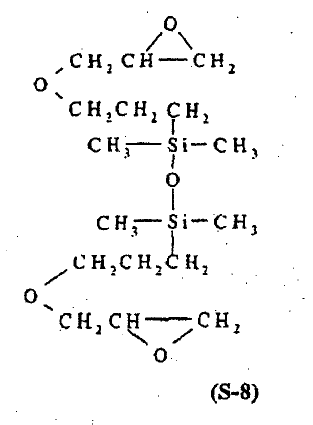
**i)** 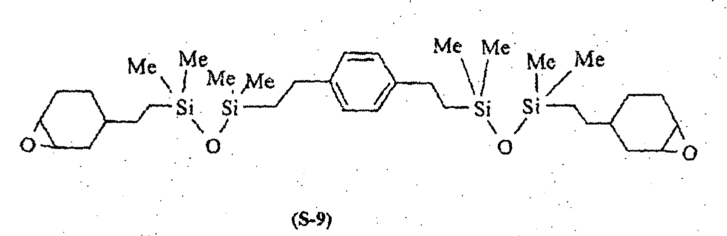
**j**) 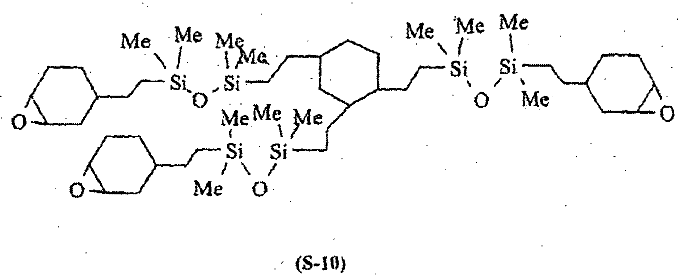
**k**) 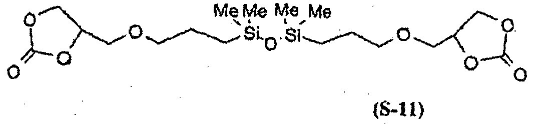
**l**) 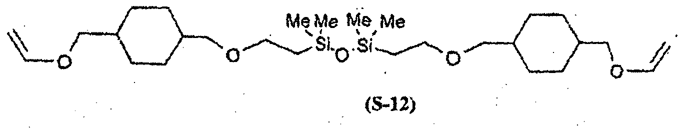
**m)** 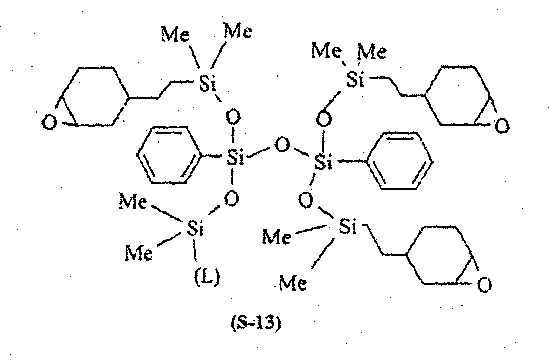 L = H; OH; Me; phenyl; C₁-C₁₂ alkyl; C₁-C₆ cycloalkyl or the following groups: 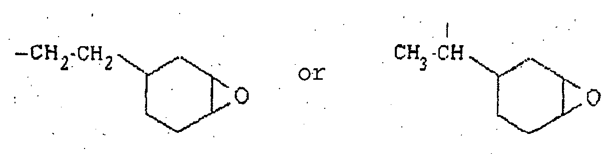
**n)** 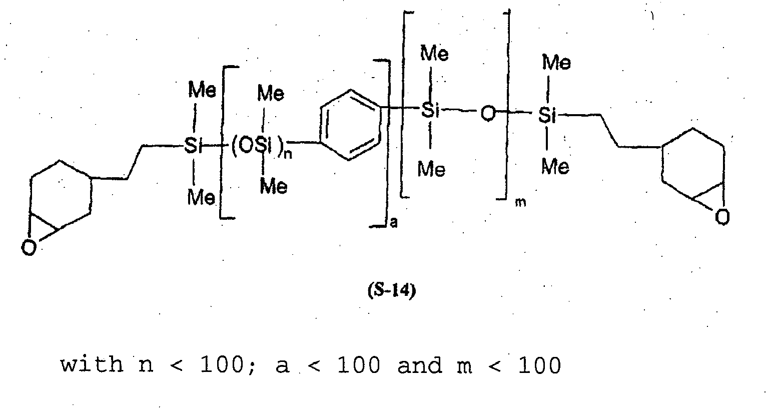
**o**) 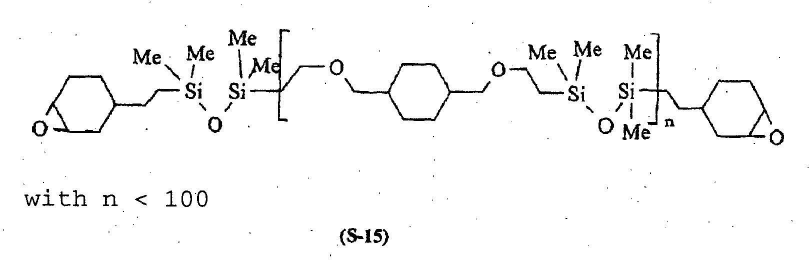
in which formulae R° or R⁰, which are identical or different, represent an alkyl, cycloalkyl or aryl radical, preferably a C₁-C₆ lower alkyl.

12. Composition according to Claim 1, **characterized in that** the cationic photoinitiator **(D)** is selected from the group consisting of the following compounds:
(**P-16**): [(C₈H₁₇)-O-C₆H₄-I-C₆H₅)]⁺, [B(C₆F₅)₄]⁻;
(**P-17**): [C₁₂H₂₅-C₆H₄-I-C₆H₅]⁺, [B(C₆F₅)₄]⁻;
(**P-18)**: [(C₈H₁₇-O-C₆H₄)₂I]⁺ [B(C₆F₅)₄]⁻;
(**P-19)**: [(C₈H₁₇)-O-C₆H₄-I-C₆H₅)]⁺, [B(C₆F₅)₄]⁻;
(**P-20**): [(C₆H₅)₂S-C₆H₄-O-C₈H₁₇]⁺ [B(C₆H₄CF₃)₄]⁻;
(**P-21**): [(C₁₂H₂₅-C₆H₄)₂I]⁺ [B(C₆F₅)₄]⁻;
(**P-22**): [CH₃-C₆H₄-I-C₆H₄-CH(CH₃)₂]⁺ [B(C₆F₅)₄]⁻;
(**P-23**): (η5-cyclopentadienyl)(η6-toluene)Fe⁺, [B(C₆F₅)₄]⁻;
(P-24): (η5-cyclopentadienyl)(η6-1-methylnaphthalene) Fe⁺, [B(C₆F₅)₄]⁻;
(P-25): (η5-cyclopentadienyl)(η6-cumene)Fe⁺, [B(C₆F₅) ₄]⁻;
(**P-26**): [(C₁₂H₂₅-C₆H₄)₂I]⁺, [B(C₆H₃(CF₃)₂]⁻;
(**P-27**): [CH₃-C₆H₄-I-C₆H₄-CH₂CH(CH₃)₂]⁺ [B(C₆F₅)₄]⁻;
(**P-28**): [CH₃-C₆H₄-I-C₆H₄-CH₂CH(CH₃)₂]⁺, [B(C₆H₃(CF₃)₂)₄]⁻; and
**(P-29):** [CH₃-C₆H₄-I-C₆H₄-CH(CH₃)₂]⁺, [B(C₆H₃(CF₃)₂)₄]⁻.

13. Dental composition according to Claim 1, **characterized in that** the cationic photoinitiator **(D)** is an iodonium salt.

14. Dental composition according to Claim 1, wherein the photosensitizer **(E)** comprises in its structure one or more substituted or unsubstituted aromatic nuclei, having a residual absorption for light of between 200 and 500 nm.

15. Dental composition according to Claim 1, wherein the photosensitizer **(E)** is selected from the group consisting of the compounds from the class of the anthracenes, thioxanthones, camphorquinones and phenanthrenequinone and also mixtures thereof.

16. Dental composition according to any one of the preceding claims, wherein the dental filler **(B)** is an inorganic glass or a fumed silica.

17. Process for treating a dental filler **(B),** comprising the following steps:
a) in a solvent medium the dental filler **(B)** and at least one organosilicon coupling agent **(F)** as defined in Claim 1 are mixed,
b) the solvent is removed by filtration to give an intermediate dental filler **(B-1),**
c) the intermediate dental filler **(B-1)** undergoes a heat treatment to terminate the coupling reaction between the intermediate dental filler **(B-1)** and the coupling agent **(F)** and so to obtain an intermediate dental filler **(B-2),**
d) the intermediate dental filler **(B-2)** is then mixed in a solvent medium with at least one compound **(G)** as defined in Claim 1 and in the presence of at least one dispersant **(H)** as defined in Claim 1, 2 or 3,
e) the solvent is removed by filtration to give an intermediate dental filler **(B-3),** and
f) the intermediate dental filler **(B-3)** undergoes a heat treatment to terminate the reaction between the intermediate dental filler **(B-3)** and the compound **(G)** and so to obtain a treated dental filler (**B**).

18. Process for treating a dental filler **(B),** comprising the following steps:
a) in a solvent medium the dental filler **(B)** and at least one organosilicon coupling agent **(F)** as defined in Claim 1 are mixed,
b) the solvent is removed by filtration to give an intermediate dental filler (**B-1**),
c) the intermediate dental filler (**B-1**) undergoes a heat treatment to terminate the coupling reaction between the intermediate dental filler **(B-1)** and the coupling agent **(F)** and so to obtain an intermediate dental filler **(B-2),**
d) the intermediate dental filler **(B-2)** is then mixed in a solvent medium with at least one compound **(G)** as defined in Claim 1,
e) the solvent is removed by filtration to give an intermediate dental filler **(B-3),**
f) the intermediate dental filler **(B-3)** undergoes a heat treatment to terminate the reaction between the intermediate dental filler **(B-3)** and the compound **(G)** and so to obtain an intermediate filler **(B-5),**
g) the intermediate dental filler **(B-5)** is then mixed in a solvent medium with at least one compound **(G)** as defined in Claim 1 and in the presence of at least one dispersant (**H**) as defined in Claim 1, 2 or 3,
h) the solvent is removed by filtration to give an intermediate dental filler **(B-6),** and
i) the intermediate dental filler **(B-6)** undergoes a heat treatment to terminate the reaction between the intermediate dental filler **(B-6)** and the compound **(G)** and so to obtain a treated dental filler **(B).**

19. Dental filler obtainable by the process according to Claim 17 or 18.

20. Use of a dental filler according to Claim 19 in dental compositions.

21. Use of a dental composition according to any one of Claims 1 to 16 for producing dental prostheses or for dental restoration.

22. Dental prosthesis obtainable by crosslinking a composition according to any one of Claims 1 to 16.

23. Dental restoration material obtainable by crosslinking a composition according to any one of Claims 1 to 16.

## Patentansprüche

1. Dentalzusammensetzung, umfassend:
(1) mindestens eine kationisch reaktive Verbindung **(A),**
(2) mindestens einen Dentalfüllstoff **(B),**
(3) gegebenenfalls mindestens einen Dentalfüllstoff (**B'**),
(4) gegebenenfalls mindestens ein Dispersionsmittel **(C),** das mindestens ein organisches Polymer oder Copolymer umfasst;
(5) mindestens einen kationischen Photostarter **(D)** und
(6) gegebenenfalls mindestens einen Photosensibilisator **(E),**
wobei die Zusammensetzung **dadurch gekennzeichnet ist, dass** der Dentalfüllstoff **(B)** vorher in Lösungsmittelmedium durch das Inkontaktbringen mit den folgenden Bestandteilen (a), (b) und (c) behandelt wird:
(a) mindestens einer Verbindung **(G),** die:
- ein organisches Monomer, Oligomer oder Polymer, oder ein Organosiloxan-Monomer, -Oligomer oder
- Polymer ist,
wobei die Verbindung **(G)** mindestens eine reaktive Oxiran-, Oxetan-, Alkenylether- und/oder Carbonatfunktion **(frC)** umfasst,
(b) mindestens einem siliciumorganischen Kopplungsmittel **(F),** das mindestens eine reaktive Funktion **(frA)** umfasst, die direkt mit einem Siliciumatom verbunden ist, die mit dem Dentalfüllstoff **(B)** reagieren kann-, und mindestens eine reaktive Funktion **(frB),** die nicht direkt an ein Siliciumatom gebunden ist, die mit einer reaktiven Funktion (**frC**) der Verbindung **(G)** reagieren kann, und
(c) mindestens einem Dispersionsmittel **(H),** das es ermöglicht, den Dentalfüllstoff **(B)** während und/oder nach der Behandlung in Lösungsmittelmedium in nicht agglomerierter Form zu erhalten.

2. Dentalzusammensetzung nach Anspruch 1, wobei das Dispersionsmittel **(H)** ein organisches Polymer oder Copolymer oder ein Monodiester der Carbonsäure ist.

3. Dentalzusammensetzung nach .Anspruch 1, wobei das Dispersionsmittel **(H)** ausgewählt ist- aus der Gruppe, bestehend aus:
- Acrylpolymeren, die gegebenenfalls durch ein Alkylammonium in ein Salz überführt sind, Polyestern, Polyethern, Polyurethanen, modifizierten Polyurethanen, Polyolpolyacrylaten,
- deren Copolymeren,
- den Monodiestern von Carbonsäuren,
- Polyurethan/Acrylatcopolymeren, die gegebenenfalls durch ein Alkylammonium in ein Salz überführt sind, und
- deren Gemische.

4. Dentalzusammensetzung nach einem der Ansprüche 1 bis 3, wobei mindestens ein Dentalfüllstoff **(B)** mit einem Verfahren **(I)** behandelt wird, das die folgenden Schritte umfasst:
a) Mischen in Lösungsmittelmedium des Dentalfüllstoffs **(B)** und mindestens eines siliciumorganischen Kopplungsmittels **(F),** wie nach Anspruch 1 definiert,
b) Entfernen des Lösungsmittels durch Filtration, um einen intermediären. Dentalfüllstoff **(B-1)** zu erhalten,
c) der intermediäre Dentalfüllstoff **(B-1)** wird einer Wärmebehandlung unterzogen, um die Kopplungsreaktion zwischen dem intermediären Dentalfüllstoff **(B-1)** und dem Kopplungsmittel **(F)** zu beenden und so einen intermediären Dentalfüllstoff **(B-2)** zu erhalten,
d) der intermediäre Dentalfüllstoff **(B-2)** wird anschließend in Lösungsmittelmedium mit mindestens einer Verbindung **(G),** wie nach Anspruch 1 definiert, und in Gegenwart mindestens eines Dispersionsmittels **(H),** wie nach Anspruch 1, 2 oder 3 definiert, gemischt,
e) Entfernen des Lösungsmittels durch Filtration, um einen intermediären Dentalfüllstoff **(B-3)** zu erhalten, und
f) der intermediäre Dentalfüllstoff **(B-3)** wird einer Wärmebehandlung unterzogen, um die. Reaktion zwischen dem intermediären Dentalfüllstoff **(B-3)** und der Verbindung **(G)** zu beenden, und so einen behandelten Dentalfüllstoff (B) zu erhalten.

5. Dentalzusammensetzung nach einem der Ansprüche 1 bis 3, wobei mindestens ein Dentalfüllstoff **(B)** mit einem Verfahren (**II**) behandelt wird, das die folgenden Schritte umfasst:
a) Mischen in Lösungsmittelmedium des Dentalfüllstoffs **(B)** und mindestens eines siliciumorganischen Kopplungsmittels **(F),** wie nach Anspruch 1 definiert,
b) Entfernen des Lösungsmittels durch Filtration, um einen intermediären Dentalfüllstoff **(B-1)** zu erhalten,
c) der intermediäre Dentalfüllstoff **(B-1)** wird einer Wärmebehandlung unterzogen, um die Kopplungsreaktion zwischen dem intermediären Dentalfüllstoff **(B-1)** und dem Kopplungsmittel **(F)** zu beenden und so einen intermediären Dentalfüllstoff **(B-2)** zu erhalten,
d) der intermediäre Dentalfüllstoff **(B-2)** wird anschließend in Lösungsmittelmedium mit mindestens einer Verbindung **(G),** wie nach Anspruch 1 definiert, gemischt,
e) Entfernen des Lösungsmittels durch Filtration, um einen intermediären Dentalfüllstoff **(B-3)** zu erhalten, und
f) der intermediäre Dentalfüllstoff **(B-3)** wird einer Wärmebehandlung unterzogen, um die Reaktion zwischen dem intermediären Dentalfüllstoff **(B-3)** und der Verbindung **(G)** zu beenden, und so einen intermediären Dentalfüllstoff **(B-5) zu** erhalten,
g) der intermediäre Dentalfüllstoff (**B-5**) wird anschließend in Lösungsmittelmedium mit mindestens einer Verbindung **(G),** wie nach Anspruch 1 definiert, und in Gegenwart mindestens eines Dispersionsmittels **(H),** wie nach Anspruch 1, 2 oder 3 definiert, gemischt,
h) Entfernen des Lösungsmittels durch Filtration, um einen intermediären Dentalfüllstoff **(B-6)** zu erhalten, und
i) der intermediäre Dentalfüllstoff **(B-6)** wird einer Wärmebehandlung unterzogen, um die Reaktion zwischen dem intermediären Dentalfüllstoff **(B-6)** und der Verbindung **(G)** zu beenden, und so einen behandelten Dentalfüllstoff **(B)** zu erhalten.

6. Dentalzusammensetzung nach Anspruch 4 oder 5, wobei die Wärmebehandlungen der, Schritte c), f) und i) der Verfahren **(I)** und **(II)** durch eine Erwärmung auf eine Temperatur kleiner oder gleich 200°C, bevorzugt kleiner oder gleich 165°C und noch stärker bevorzugt im Bereich zwischen 100 und 165°C durchgeführt werden.

7. Dentalzusammensetzung nach Anspruch 1, wobei:
- die reaktive Funktion **(frA),** die direkt an ein Siliciumatom des siliciumorganischen Kopplungsmittels **(F)** gebunden ist, eine Alkoxy-, Enoxy- oder Hydroxyfunktion ist;
- die reaktive Funktion **(frB),** die nicht direkt an ein Siliciumatom des siliciumorganischen Kopplungsmittels **(F)** gebunden ist, eine Oxiran-, Oxetan-, Hydroxy-, Säure-, Carbonsäureanhydrid- oder Diolfunktiön ist; und
- wobei die reaktive Funktion **(frC)** der Verbindung **(G)** eine Oxiran-, Oxetan-, Alkenylether- oder Carbonatfunktion ist.

8. Dentalzusammensetzung nach einem der vorhergehenden Ansprüche, wobei das siliciumorganische Kopplungsmittel **(F)** ausgewählt ist aus der Gruppe bestehend aus den folgenden Verbindungen: Glycidyloxypropyltrimethoxysilan, dem Hydrolyseprodukt von Glycidyloxypropyltrimethoxysilan; Glycidyloxypropyltriethoxysilan, dem Hydrolyseprodukt von Glycidyloxypropyltriethoxysilan in saurem Medium; Glycidyloxypropyldimethoxymethylsilan oder dem Hydrolyseprodukt, beta-(3,4-Epoxycyclohexyl)ethyltriethoxysilan oder dem Hydrolyseprodukt und beta-(3,4-Epoxycyclohexyl)ethyltrimethoxysilan oder dem Hydrolyseprodukt.

9. Dentalzusammensetzung nach einem der vorhergehenden Ansprüche, wobei die Verbindung **(G)** ein Silikonoligomer **(G-1)** oder ein Silikonpolymer **(G-2)** ist, das mindestens eine reaktive Öxiran-, Oxetan-, Alkenylether- und/oder Carbonatfunktion **(frC)** umfasst.

10. Dentalzusammensetzung nach Anspruch 9, wobei das Silikonoligomer **(G-1)** oder Silikonpolymer **(G-2),** Folgendes umfasst:
a) mindestens eine Struktur der Formel: 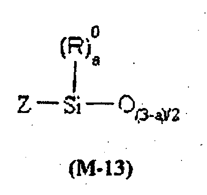 wobei in der Formel:
- a = 0, 1 oder 2,
- R⁰, gleich oder verschieden, für einen Alkyl-, Cycloalkyl-, Aryl-, Vinyl-, Wasserstoff-, Alkoxyrest, bevorzugt für einen Alkylrest mit weniger als 1 bis 6 C-Atomen steht,
- Z, gleich oder verschieden, ein organischer Substituent ist, der mindestens eine Oxiran-, Alkenylether-, Oxetan- und/oder Carbonatfunktion umfasst, und
b) mindestens zwei Siliciumatome.

11. Dentalzusammensetzung nach Anspruch 10, wobei das Silikonoligomer **(G-1)** oder das Silikonpolymer **(G-2)** ausgewählt ist aus der Gruppe, bestehend aus den Verbindungen der folgenden Formeln **(S-1)** bis **(S-15):**
**a)** 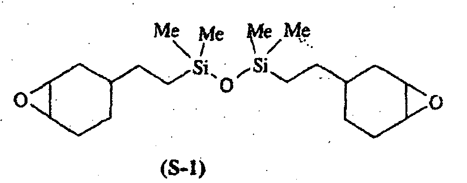
**b)** 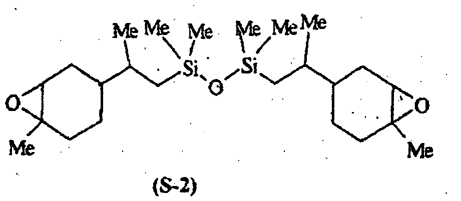
**c)** 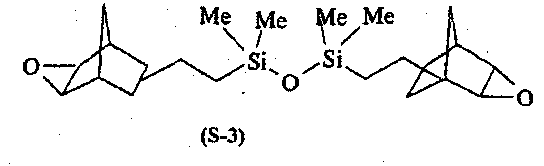
**d)** 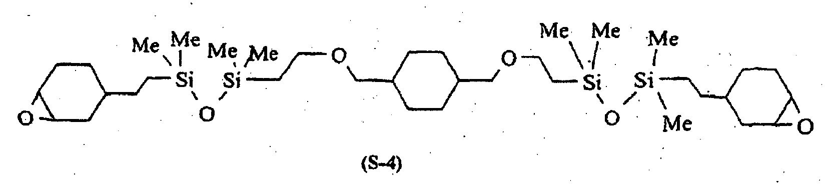
**e)** 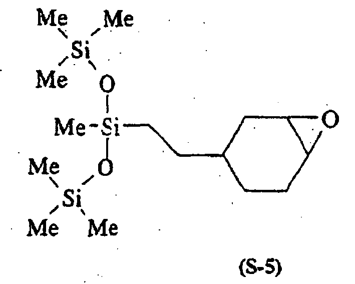
**f)** 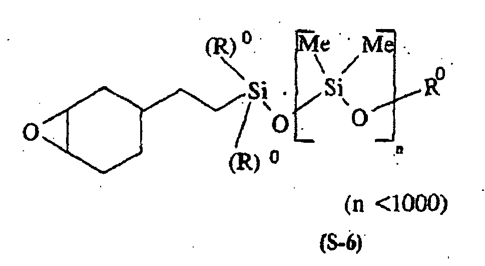
**g)** 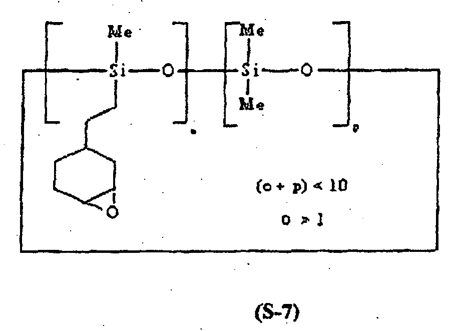
**h)** 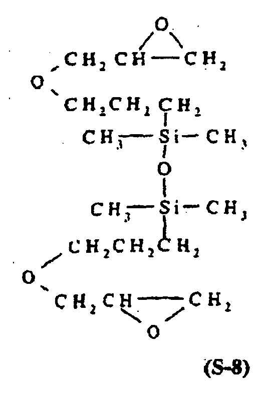
**i)** 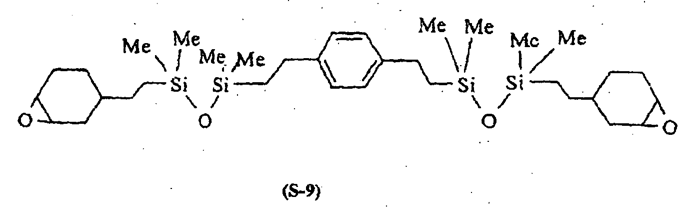
**j)** 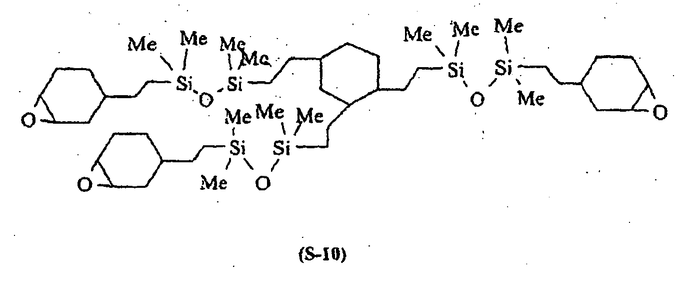
**k)** 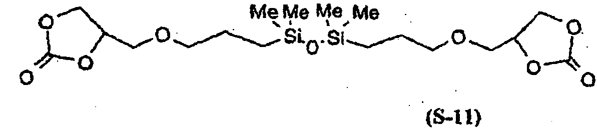
**l)** 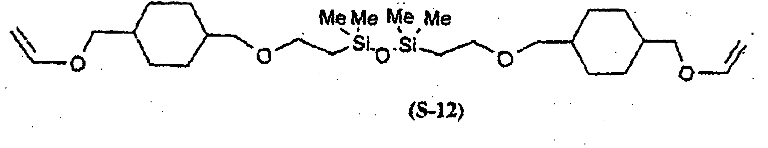
**m)** 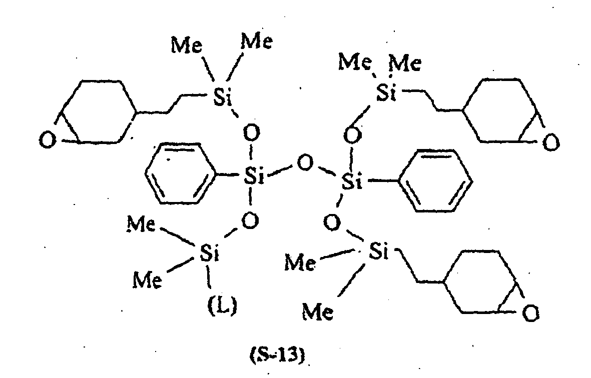 L = H; OH; Me; Phenyl; C₁-C₁₂-Alkyl; C₁-C₆-Cycloalkyl oder die folgenden Gruppen: 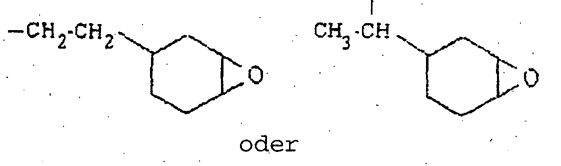
**n)** 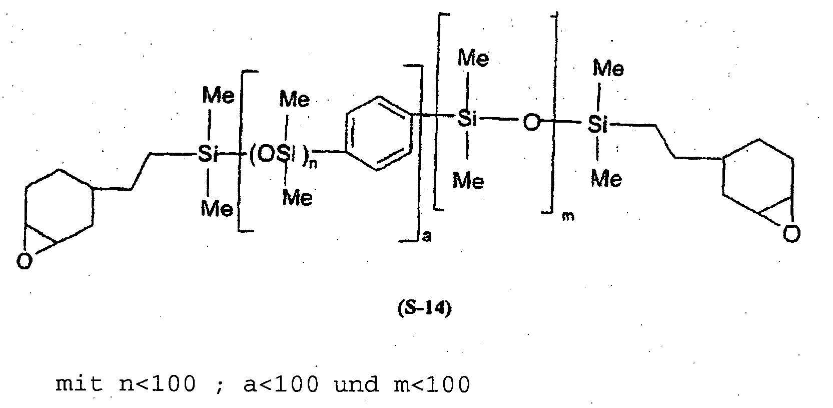
**o)** 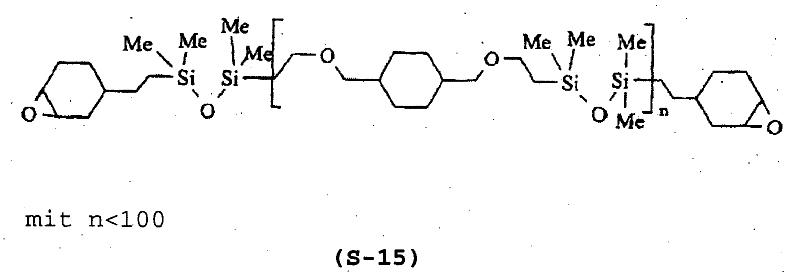
wobei in den Formeln R⁰ oder R₀, gleich oder verschieden, für einen Alkyl-, Cycloalkyl-, Arylrest, bevorzugt einen Alkylrest mit weniger als 1 bis 6 C-Atomen, steht.

12. Zusammensetzung nach Anspruch 1, **dadurch gekennzeichnet, dass** der kationische Photostarter **(D)** ausgewählt ist aus der Gruppe, bestehend aus den folgenden Verbindungen:
**(P-16):** [(C₈H₁₇)-O- C₆H₄-I-C₆H₅)]⁺, [B(C₆F₅)₄]⁻;
**(P-17):** [C₁₂H₂₅-C₆H₄-I-C₆H₅]⁻, [B(C(F₅)₄]⁻;
**(P-18):** [(C₈H₁₇-O- C₆H₄)₂I]⁺, [B(C₆F₅)₄]⁻;
**(P-19):** [(C₈H₁₇)-O-C₆H₄-I-C₆H₅)]⁻, [B(C₆F₅)₄]⁻;
**(P-20):** [(C₆H₅)₂S-C₆H₄-O-C₈H₁₇]⁻, [B (C₆H₄CF₃)₄]⁻;
**(P-21):** [(C₁₂H₂₅-C₆H₄]₂I]⁻, [B(C₆F₅)₄]⁻;
**(P-22):** [CH₃-C₆H₄-I-C₆H₄-CH(CH3)₂]⁻ [B(C₆F₅)₄]⁻;
**(P-23):** (η5-Cyclopentadienyl) (η6-Toluen) Fe⁻, [B(C₆F₅)₄]⁻;
**(P-24):** (η5-Cyclopentadienyl) (η6-Methyl-1-naphthalen) Fe⁻, [B(C₆F₅)₄]⁻;
**(P-25):** (η5-Cyclopentadienyl) (η6-Cumen) Fe⁺, [B(C₆F₅)₄]⁻;
**(P-26):** [(C₁₂H₂₅-C₆H₄)₂I]⁺, [B(C₆H₃(CF₃)₂]⁻;
**(P-27):** [CH₃C₆H₄I -C₆H₄-CH₂CH(CH₃)₂]⁻, [B(C₆F₅)₄]⁻;
**(P-28):** [CH₃-C₆H₄-I-C₆H₄-CH₂CH (CH₃)₂]+, [B(C₆H₃(CF₃)₂)₄]⁻ und
**(P-29):** [CH₃-C₆H₄-I-C6H4-CH(CH₃)₂]⁺, [B(C₆H₃(CF)₂)₄]⁻.

13. Dentalzusammensetzung nach Anspruch 1, **dadurch gekennzeichnet, dass** der kationische Photostarter **(D)** ein Iodoniumsalz ist.

14. Dentalzusammensetzung nach Anspruch 1, wobei der Photosensibilisator **(E)** in seiner Struktur einen oder mehrere aromatische Zentren, substituiert oder nicht, umfasst, die eine Restabsorption des Lichts im Bereich zwischen 200 und 500 nm aufweisen.

15. Dentalzusammensetzung nach Anspruch 1, wobei der Photosensibilisator **(E)** ausgewählt ist aus der Gruppe bestehend aus den Verbindungen der Klasse der Anthracene, Thioxanthone; Campherchinone und Phenanthrenchinon, sowie deren Gemischen.

16. Dentalzusammensetzung nach einem der vorhergehenden Ansprüche, wobei der. Dentalfüllstoff **(B)** ein mineralisches Glas oder eine pyrogene Kieselsäure ist.

17. Verfahren zum Behandeln eines Dentalfüllstoffs **(B),** das die folgenden Schritte umfasst:
a) Mischen in Lösungsmittelmedium des Dentalfüllstoffs **(B)** und mindestens eines siliciumorganischen Kopplungsmittels **(F),** wie nach Anspruch 1 definiert,
b) Entfernen des Lösungsmittels durch Filtration, um einen intermediären Dentalfüllstoff **(B-1)** zu erhalten,
c) der intermediäre Dentalfüllstoff **(B-1)** wird einer Wärmebehandlung unterzogen, um die Kopplungsreaktion zwischen dem intermediären. Dentalfüllstoff **(B-1)** und dem Kopplungsmittel **(F)** zu beenden und so einen intermediären Dentalfüllstoff **(B-2)** zu erhalten,
d) der intermediäre Dentalfüllstoff **(B-2)** wird anschließend in Lösungsmittelmediüm mit mindestens einer Verbindung. **(G),** wie nach Anspruch 1 definiert, und in Gegenwart mindestens eines Dispersionsmittels **(H),** wie nach Anspruch 1, 2 oder 3 definiert, gemischt,
e) Entfernen des Lösungsmittels durch Filtration, um einen intermediären Dentalfüllstoff **(B-3)** zu erhalten, und
f) der intermediäre Dentalfüllstoff **(B-3)** wird einer Wärmebehandlung unterzogen, um die Reaktion zwischen dem intermediären Dentalfüllstoff **(B-3)** und der Verbindung **(G)** zu beenden, und so einen behandelten Dentalfüllstoff **(B)** zu erhalten.

18. Verfahren zum Behandeln eines Dentalfüllstoffs **(B),** das die folgenden Schritte umfasst:
a) Mischen in Lösungsmittelmedium des Dentalfüllstoffs **(B)** und mindestens eines siliciumorganischen Kopplungsmittels **(F),** wie nach Anspruch 1 definiert,
b) Entfernen des Lösungsmittels durch Filtration, um einen intermediären Dentalfüllstoff **(B-1)** zu erhalten,
c) der intermediäre Dentalfüllstoff **(B-1)** wird einer Wärmebehandlung unterzogen, um die Kopplungsreaktion zwischen dem intermediären Dentalfüllstoff **(B-1)** und dem Kopplungsmittel **(F)** zu beenden und so einen intermediären Dentalfüllstoff **(B-2)** zu erhalten,
d) der intermediäre Dentalfüllstoff **(B-2)** wird anschließend in Lösungsmittelmedium mit mindestens einer Verbindung **(G),** wie nach Anspruch 1 definiert, gemischt,
e) Entfernen des Lösungsmittels durch Filtration, um einen intermediären Dentalfüllstoff **(B-3)** zu erhalten, und
f) der intermediäre Dentalfüllstoff **(B-3)** wird einer Wärmebehandlung unterzogen, um die Reaktion zwischen dem intermediären Dentalfüllstoff **(B-3)** und der Verbindung **(G)** zu beenden, und so einen intermediären Dentalfüllstoff **(B-5)** zu erhalten,
g) der intermediäre Dentalfüllstoff **(B-5)** wird anschließend in Lösungsmittelmedium mit mindestens einer Verbindung **(G),** wie nach Anspruch 1 definiert, und in Gegenwart mindestens eines Dispersionsmittels **(H),** wie nach Anspruch 1, 2 oder 3 definiert, gemischt,
h) Entfernen des Lösungsmittels durch Filtration, um einen intermediären Dentalfüllstoff **(B-6)** zu erhalten, und
i) der intermediäre Dentalfüllstoff **(B-6)** wird einer Wärmebehandlung unterzogen, um die Reaktion zwischen dem intermediären Dentalfüllstoff **(B-6)** und der Verbindung **(G)** zu beenden und so einen behandelten Dentalfüllstoff **(B)** zu erhalten.

19. Dentalfüllstoff, der durch das Verfahren nach Anspruch 17 oder 18 erhalten werden kann.

20. Verwendung eines Dentalfüllstoffs nach Anspruch 19 in Dentalzusammensetzungen.

21. Verwendung, einer Dentalzusammensetzung nach einem der Ansprüche 1 bis 16 zur Herstellung von Dentalprothesen oder zur Dentalrestauration.

22. Dentalprothese, die durch Vernetzung einer Zusammensetzung nach einem der Ansprüche 1 bis 16 erhalten werden kann.

23. Material zur Dentalrestauration, das durch Vernetzung einer Zusammensetzung nach einem der Ansprüche 1 bis 16 erhalten werden kann.
